(19) Europäisches Patentamt
European Patent Office
Office européen des brevets

(11) EP 2 298 738 B1

(12) **EUROPEAN PATENT SPECIFICATION**

(45) Date of publication and mention of the grant of the patent:
**19.09.2012 Bulletin 2012/38**

(21) Application number: **10177536.9**

(22) Date of filing: **31.10.2001**

(51) Int Cl.:
***C07D 209/08*** *(2006.01)*
***C07D 231/56*** *(2006.01)*
***C07D 403/12*** *(2006.01)*
***C07D 409/12*** *(2006.01)*
***C07D 417/12*** *(2006.01)*

(54) **1-aryl- or 1-alkylsulfonyl-heterocyclylbenzazoles as 5-hydroxytryptamine-6 ligands**

1-Aryl- oder 1-Alkylsulfonyl Heterocyclylbenzazole als 5-Hydroxytryptamin-6-Liganden

1-aryl- ou 1-alkylsulfonyl-heterocyclylbenzazoles en tant que ligands de 5-hydroxytryptamine-6

(84) Designated Contracting States:
**AT BE CH CY DE DK ES FI FR GB GR IE IT LI LU MC NL PT SE TR**
Designated Extension States:
**AL LT LV MK RO SI**

(30) Priority: **02.11.2000 US 245118 P**

(43) Date of publication of application:
**23.03.2011 Bulletin 2011/12**

(62) Document number(s) of the earlier application(s) in accordance with Art. 76 EPC:
**01992697.1 / 1 343 756**

(73) Proprietor: **Wyeth LLC**
**Madison, NJ 07940 (US)**

(72) Inventors:
- **Kelly, Michael, Gerard**
**Thousand Oaks, CA 91360 (US)**
- **Cole, Derek, Cecil**
**New City, NY 10956 (US)**

(74) Representative: **Pfizer**
**European Patent Department**
**23-25 avenue du Docteur Lannelongue**
**75668 Paris Cedex 14 (FR)**

(56) References cited:
**WO-A1-96/03400**

- **ISAAC M ET AL: "6-bicyclopiperazinyl-1-arylsulfonylindole s and 6-bicyclopiperidinyl-1-arylsulfonylindoles derivatives as novel, potent, and selective 5-HT6 receptor antagonists", BIOORGANIC & MEDICINAL CHEMISTRY LETTERS, PERGAMON, ELSEVIER SCIENCE, GB, vol. 10, no. 15, 7 August 2000 (2000-08-07), pages 1719-1721, XP004213231, ISSN: 0960-894X, DOI: DOI:10.1016/S0960-894X(00) 00320-6**

EP 2 298 738 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to 1-Aryl- or 1-alkylsulfonyl-heterocyclylbenzazoles useful as 5-hydroxytryptamine-6 ligands, to processes for preparing them, to pharmaceutical compositions containing them and to methods of treatment using them.

## BACKGROUND OF THE INVENTION

Compounds capable of forming 5-HT6 receptor ligands are potentially useful in the treatment of a number of central nervous system disorders such as anxiety, depression, epilepsy obsessive compulsive disorders, migraine, cognitive disorders, sleep disorders, feeding disorders, panic attacks, disorders resulting from withdrawal from drug abuse, schizophrenia, or certain gastrointestinal disorders such as irritable bowel syndrome. Significant efforts are being made to understand the recently identified 5HT-6 receptor and its possible role in neuropsychiatric and neurodegenerative functions. To that end, new compounds which demonstrate a binding affinity for the 5HT-6 receptor are earnestly sought, particularly as potential potent therapeutic agents.

Therefore, it is an object of this invention to provide compounds which are useful as therapeutic agents in the treatment of a variety of conditions related to or affected by the 5-HT6 receptor.

It is another object of this invention to provide methods and compositions useful for the treatment of psychoses (e.g., schizophrenia, anxiety, or depression), motor disorders (e.g., Parkinson's disease), anxiety, depression, obsessive compulsive disorder, attention deficit disorder, or any condition which is known to be related to or affected by the 5-HT6 receptor.

These and other objects and features of this invention will become more apparent by the detailed description set forth hereinbelow.

## SUMMARY OF THE INVENTION

The present invention provides a compound of formula I

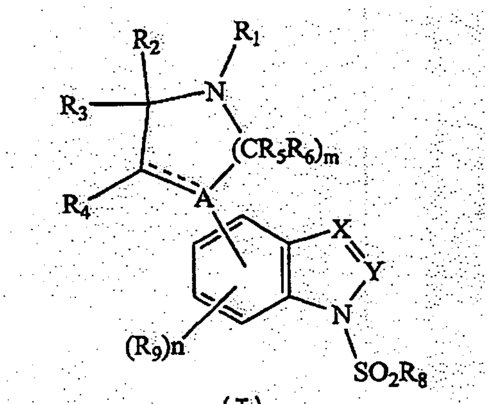

(I)

wherein
A is C, Crit or N;
X is N;
Y is $CR_7$;
$R_1$ is H, $C_1$-$C_6$alkylcarbonyl, $C_1$-$C_6$alkoxycarbonyl or an C1-$C_6$alkyl, $C_2$-$C_6$alkenyl, $C_2$-$C_6$alkynyl or $C_5$-$C_7$cycloheteroalkyl group each optionally substituted;
$R_2$, $R_3$, $R_4$, $R_5$ and $R_6$ are each independently H, halogen, OH or an optionally substituted $C_1$-$C_6$alkyl group;
$R_7$ is H, halogen or a $C_1$-$C_6$alkyl, aryl, heteroaryl or $C_1$-$C_6$alkoxy group each optionally substituted;
$R_8$ is a $C_1$-$C_6$alkyl, aryl or heteroaryl group each optionally substituted;
$R_9$ is H, halogen or a $C_1$-$C_6$alkyl, $C_1$-$C_6$alkoxy, $C_2$-$C_6$alkenyl, aryl or heteroaryl group each optionally substituted;
$R_{10}$ is H, OH or an optionally substituted alkoxy group
m is an integer of 1, 2 or 3;
n is 0 or an integer of 1, 2 or 3; and
---- represents a single bond or a double bond; or a pharmaceutically acceptable salt thereof.

The present invention also provides methods and compositions useful in the treatment of central nervous system disorders.

## DETAILED DESCRIPTION OF THE INVENTION

The 5-hydroxytryptamine-6 (5-HT6) receptor is one of the most recent receptors to be identified by molecular cloning. Its ability to bind a wide range of therapeutic compounds used in psychiatry, coupled with its intriguing distribution in the brain has stimulated significant interest in new compounds which are capable of interacting with or affecting said receptor. At present, there are no known fully selective agonists. Significant efforts are being made to understand the possible role of the 5-HT6 receptor in psychiatry, cognitive dysfunction, motor function and control, memory, mood and the like. To that end, compounds which demonstrate a binding affinity for the 5-HT6 receptor are earnestly sought both as an aid in the study of the 5-HT6 receptor and as potential therapeutic agents in the treatment of central nervous system disorders.

Surprisingly, it has now been found that 1-alkyl- or 1-arylsulfonyl-heterocyclylbenzazoles of formula I demonstrate 5-HT6 affinity along with significant sub-type selectivity. Advantageously, said formula I benzazoles are effective therapeutic agents for the treatment of central nervous system disorders associated with or affected by the 5-HT6 receptor. Accordingly, the present invention provides 1-alkyl- or 1-arylsulfonyl-heterocyclylbenzazole compounds of formula I

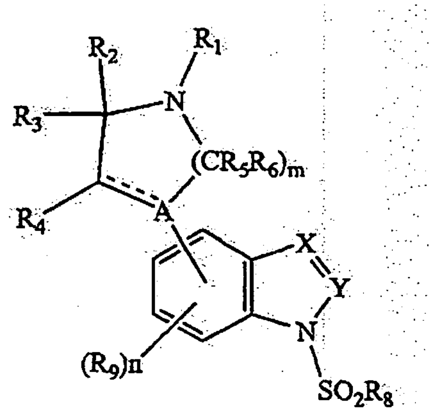

(I)

wherein
A is C, $CR_{10}$ or N;
X is N;
Y is $CR_7$ ;
$R_1$ is H, $C_1$-$C_6$alkylcarbonyl, $C_1$-$C_6$alkoxycarbonyl or a $C_1$-$C_6$alkyl, $C_2$-$C_6$alkenyl, $C_2$-$C_6$alkynyl or cycloheteroalkyl group each optionally substituted;
$R_2$, $R_3$, $R_4$, $R_5$ and $R_6$ are each independently H, halogen, OH or an optionally substituted $C_1$-$C_6$alkyl group;
$R_7$ is H, halogen or an $C_1$-$C_6$alkyl, aryl, heteroaryl or alkoxy group each optionally substituted;
$R_8$ is an $C_1$-$C_6$alkyl, aryl or heteroaryl group each optionally substituted;
$R_9$ is H, halogen or an $C_1$-$C_6$alkyl, $C_1$-$C_6$alkoxy, $C_2$-$C_6$alkenyl, aryl or heteroaryl group each optionally substituted;
$R_{10}$ is H, OH or an optionally substituted alkoxy group;
m is an integer of 1, 2 or 3;
n is O or an integer of 1, 2 or 3; and
---- represents a single bond or a double bond; or a pharmaceutically acceptable salt thereof.

As used in the specification and claims, the term halogen designates Br, Cl, I or F; the term aryl designates phenyl or naphthyl. The term cycloheteroalkyl designates a five to seven membered cycloalkyl ring system containing 1 or 2 heteroatoms, which may be the same or different, selected from N, NR, O or S and optionally containing one double bond, where R represents hydrogen or an optional substituent such as illustrated herein. Exemplary of the cycloheteroalkyl ring systems included in the term as designated herein are the following rings wherein Y is NR, O or S.

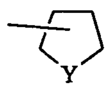 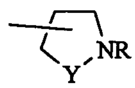 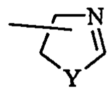 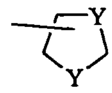 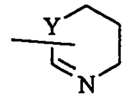

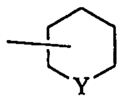 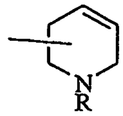 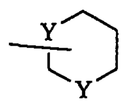 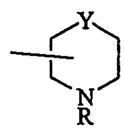 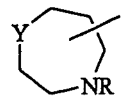

Similarly, as used in the specification and claims, the term heteroaryl designates a 5-10 membered aromatic ring system containing 1, 2 or 3 heteroatoms, which may be the same or different, selected from nitrogen, oxygen and sulphur. Such heteroaryl ring systems include pyrrolyl, azolyl, oxazolyl, thiazolyl, imidazolyl, furyl, thienyl, quinolinyl, isoquinolinyl, indolinyl, benzothienyl, benzofuranyl, benzisoxazolyl and the like; the term haloalkyl designates a $C_nH_{2n+1}$ group having from one to 2n+1 halogen atoms which may be the same or different; and the term haloalkoxy designates an $OC_nH_{2n+1}$ group having from one to 2n+1 halogen atoms which may be the same or different.

In the specification and claims, when terms such as $C_1$-$C_6$alkyl, $C_2$-$C_6$alkenyl, $C_2$-$C_6$alkynyl, $C_3$-$C_7$cycloalkyl, cycloheteroalkyl, aryl or heteroaryl are designated as being optionally substituted, the substituent groups which are optionally present may be one or more of those customarily employed in the development of pharmaceutical compounds or the modification of such compounds to influence their structure/activity, persistence, absorption, stability or other beneficial property.

Specific examples of such substituents include halogen atoms, nitro, cyano, thiocyanato, cyanato, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl, alkylamido, phenyl, phenoxy, benzyl, benzyloxy, heteroaryl, cycloheteroalkyl or cycloalkyl groups, preferably halogen atoms or lower alkyl groups. Typically, 0-3 substituents may be present. When any of the foregoing substituents represents or contains an alkyl substituent group, this may be linear or branched and may contain up to 12, preferably up to 6, more preferably up to 4 carbon atoms.

The variables A, X, Y, $R_1$, $R_2$, $R_3$, $R_4$, $R_5$, $R_6$, $R_7$, $R_8$, $R_9$, $R_{10}$ may each be values that are optionally substituted by substituents as described herein.

Examples of the variables in formula (I) are each or any combination of the following:

A is C, N, or $CR_{10}$ wherein $R_{10}$ is as defined or illustrated herein (e.g. A is CH, C(OH), C(O-$C_1$-$C_6$alkyl) wherein the alkyl group may be substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups).

Y is $CR_7$ wherein $R_7$ is as defined or illustrated herein (e.g. $CR_7$ is CH, C-aryl, C-halogen, C-($C_1$-$C_6$alkyl), C (O-$C_1$-$C_6$alkyl) wherein the alkyl or aryl groups may each be substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups).

$R_1$ is H, $C_1$-$C_6$alkylcarbonyl, $C_1$-$C_6$alkyloxycarbonyl or an $C_1$-$C_6$alkyl, $C_1$-$C_6$alkenyl, $C_1$-$C_6$alkynyl or 5-7 membered cycloheteroalkyl group each optionally substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-($C_1$-$C_6$-alkyl)amino, formyl, $C_2$-$C_7$alkoxycarbonyl, carboxyl, $C_2$-$C_7$-alkanoyl, $C_1$-$C_6$-alkylthio, $C_1$-$C_6$-alkylsulphinyl, $C_1$-$C_6$alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups); said phenyl, phenoxy, benzyl and benzyloxy groups being optionally substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-($C_1$-$C_6$-alkyl) amino, formyl, $C_2$-$C_7$alkoxycarbonyl, carboxyl, $C_2$-$C_7$-alkanoyl, $C_1$-$C_6$-alkylthio, $C_1$-$C_6$-alkylsulphinyl, $C_1$-$C_6$alkylsulphonyl, carbamoyl, $C_1$-$C_6$-alkylamido.

$R_2$, $R_3$, $R_4$, $R_5$ and $R_6$ are each selected from H, halogen OH or $C_1$-$C_6$alkyl wherein the alkyl group may be substituted by one or more of the following the same or different: halogen, nitro, cyano, thiocyanato, cyanato, hydroxyl,

$C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups).

$R_7$ is H, halogen, aryl, heteroaryl, $C_1$-$C_6$alkyl or O-$C_1$-$C_6$alkyl wherein the alkyl, aryl or heteroaryl groups may each be substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups).

$R_8$ is a $C_1$-$C_6$alkyl, aryl or heteroaryl wherein the alkyl, aryl or heteroaryl groups may each be substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups); said phenyl, phenoxy, benzyl and benzyloxy groups being optionally substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-($C_1$-$C_6$-alkyl)amino, formyl, $C_2$-$C_7$alkoxycarbonyl, carboxyl, $C_2$-$C_7$-alkanoyl, $C_1$-$C_6$-alkylthio, $C_1$-$C_6$-alkylsulphinyl, $C_1$-$C_6$alkylsulphonyl, carbamoyl, $C_1$-$C_6$-alkylamido.

$R_9$ is H, halogen, aryl, heteroaryl, $C_2$-$C_6$alkenyl, $C_1$-$C_6$alkyl or O-$C_1$-$C_6$alkyl wherein the alkenyl, alkyl, aryl or heteroaryl groups may each be substituted by one or more of the following the same or different: halogen, nitro, cyano, thiocyanato, cyanato, hydroxyl, $C_1$-$C_6$-alkyl, $C_1$-$C_{6-}$alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups).

$R_{10}$ is H, OH or O-$C_1$-$C_6$alkyl wherein the alkyl, group may be substituted by one or more of the following the same or different: halogen, nitro, cyano, thiocyanato; cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_{6-}$alkylsulphonyl, carbamoyl, $C_1$-$C_6$-alkylamido, phenyl, phenoxy, benzyl, benzyloxy, heteroaryl, cycloheteroalkyl or $C_5$-$C_7$cycloalkyl groups).

More particularly, independent examples of the variables in formula (I) are each of the following:

A may represent N, CH, C(OH), C (O-$C_1$-$C_6$alkyl) wherein the alkyl group may be substituted by one or more of the following the same or different: halogen, hydroxyl, $C_1$-$C_6$-alkoxy, amino, $C_1$-$C_6$-alkylamino, di-$C_1$-$C_6$-alkylamino and phenyl.

Y may represent CH, C-aryl, C-halogen, C-($C_1$-$C_6$alkyl) or C(O-$C_1$-$C_6$alkyl).

$R_1$ may represent H, ($C_1$-$C_6$alkyl)carbonyl, $C_5$-$C_7$-cycloheteroalkyl having 1 or 2 nitrogen ring atoms, or an $C_1$-$C_6$ alkyl, phenyl$C_1$-$C_6$ alkyl, pyridyl$C_1$-$C_6$alkyl, thienyl$C_1$-$C_6$alkyl group each optionally substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-($C_1$-$C_6$-alkyl)amino, formyl, $C_2$-$C_7$alkoxycarbonyl, carboxyl, $C_2$-$C_7$-alkanoyl, $C_1$-$C_6$-alkylthio, $C_1$-$C_6$-alkylsulphinyl, $C_1$-$C_6$alkylsulphonyl, carbamoyl, $C_1$-$C_6$-alkylamido, phenyl, phenoxy, benzyl, benzyloxy, cycloheteroalkyl or $C_5$-$C_7$cycloalkyl groups.

$R_2$, $R_3$, $R_4$, $R_5$ and $R_6$ may each independently represent H, halogen, OH or -$C_1$-$C_6$alkyl.

$R_8$ may represent a $C_1$-$C_6$alkyl, aryl of 6-10 carbon atoms or mono- or bi-cyclic heteroaryl 6-10 carbon atoms or heteroaryl of 5-10 ring members having 1-3 heteroatoms selected from O, N and S wherein the aryl or heteroaryl groups may each be substituted by one or more of the following the same or different: halogen, nitro, cyano, thiocyanato, cyanato, hydroxyl, $C_1$-$C_6$-alkyl, $C_1$-$C_6$-alkoxy, halo$C_1$-$C_6$-alkoxy, halo$C_1$-$C_6$-alkyl, amino, $C_1$-$C_6$-alkylamino, di-$C_1$-$C_6$-alkylamino, formyl, $C_2$-$C_7$-alkoxycarbonyl, carboxyl, $C_2$-$C_7$-alkanoyl, $C_1$-$C_6$-alkylthio, $C_1$-$C_6$-alkylsulphinyl, $C_1$-$C_6$-alkylsulphonyl, carbamoyl, $C_1$-$C_6$-alkylamido, phenyl, phenoxy, benzyl, benzyloxy, cycloheteroalkyl or $C_5$-$C_7$cycloalkyl groups);

$R_9$ may represent H, halogen, $C_1$-$C_6$alkyl

$R_{10}$ may represent H, OH or O-$C_1$-$C_6$alkyl.

Further examples of $R_1$ are hydrogen, $C_1$-$C_6$alkyl (e.g. propyl); ($C_1$-$C_6$alkyl) -CO- (e.g. acetyl); benzyl; phenethyl; phenpropyl; pyridylmethyl (e.g. 3- or 4-pyridylmethyl); thienylmethyl;, benzoyl($C_{1-}C_4$)alkyl, phenoxy($C_1$-$C_4$)alkyl and 4,5-dihydro-1H-imidazolyl; which groups may be substituted by one or more substituents the same or different such as substituents selected from halogen (e.g. 2-chloro-5-thienylmethyl, 2-(p-fluorophenoxy)ethyl, p-fluorobenzoylpropyl); nitro

(e.g. 3-nitrobenzyl); or ($C_1$-$C_6$)alkoxy (e.g. 3-methoxybenzyl).

Further examples of $R_8$ are phenyl, naphthyl and heteroaryl groups as hereinbefore defined such as thienyl (e.g thien-2-yl), benzothienyl (e.g benzothien-2-yl), imidazo[2,1-b]thiazolyl, benzothiazolyl, benzofurazanyl, benzothiadiazolyl, isoxazolyl, imidazolyl and pyrazolyl (e.g pyrazol-4-yl); which groups may each be substituted by one or more substituents (e.g 1-3) the same or different such as substituents selected from halogen, $C_1$-$C_4$ alkoxy, $C_1$-$C_4$ alkyl, $C_1$-$C_4$ haloalkyl, $C_1$-$C_4$ haloalkoxy $C_1$-$C_4$alkylamino, fi($C_1$-$C_4$alkyl)amino and amino.

Examples of m are 2 and 3. $R_5$ and $R_6$ may be for example hydrogen. $R_2$, $R_3$ and $R_5$ may also represent hydrogen. An example of n is zero. A may be for example -N- , -CH- or -C(OH)-.

Pharmaceutically acceptable salts may be any acid addition salt formed by a compound of formula I and a pharmaceutically acceptable acid such as phosphoric, sulfuric, hydrochloric, hydrobromic, citric, maleic, succinic, fumaric, acetic, lactic, nitric, sulfonic, p-toluene sulfonic, methane sulfonic acid or the like.

Preferred compounds of the invention are those compounds of formula I wherein A is N and m is 2. Also preferred are those compounds of formula I wherein $R_8$ is an optionally substituted phenyl group and $R_1$ is H or a $C_1$-$C_6$alkyl or $C_5$-$C_7$cycloheteroalkyl group each optionally substituted. Further preferred compounds of the invention are those compounds of formula I wherein $R_2$, $R_3$, $R_4$ , $R_5$ and $R_6$ are H and n is 0 .

More preferred compounds of the invention are those compounds of formula I wherein A is N; m is 2 and $R_1$ is H or a $C_1$-$C_4$alkyl or $C_5$-$C_7$cycloheteroalkyl group each optionally substituted. Another group of more preferred compounds of the invention are those compounds of formula I wherein A is N; m is 2 ; $R_1$ is H or a $C_1$-$C_4$alkyl or $C_5$-$C_7$cycloheteroalkyl group each optionally substituted; and $R_8$ is an optionally substituted phenyl group.

This invention also provides processes for preparing compounds of formula I which processes comprises one of the following:

i) reacting a compound of formula:

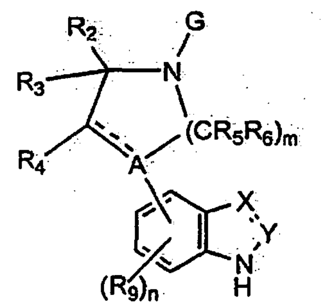

wherein the dotted line, n, m, $R_2$, $R_3$, $R_4$, $R_5$, $R_6$, $R_9$, X, Y and A are as defined above and G is a protecting group, with a suphonylating agent containing the group:

$$R_8SO_2-$$

wherein $R_8$ is as defined above, and if required removing the protecting group G to give a compound of Formula I wherein $R_1$ is hydrogen;
or

ii) reacting a compound of formula

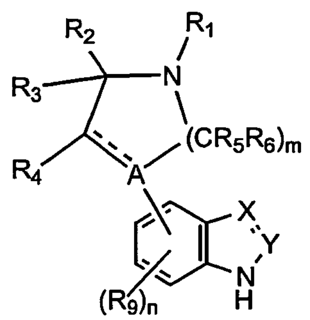

wherein the dotted line, n, m, $R_1$, $R_2$, $R_3$, $R_4$, $R_5$, $R_6$, $R_9$, X, Y and A are as defined above, with a sulphonylating agent containing the group

$R_8SO_2$-

wherein $R_8$ is as defined above, to give a compound of formula (I); or

iii) reacting a compound of formula I wherein $R_1$ is hydrogen with a compound of formula:

**$R_1$-** L

wherein $R_1$ is as defined above (excepting hydrogen) and L is a suitable leaving group, e.g. halogen or SMe to give a corresponding compound of formula I; or

iv) alkylating a compound of formula (I) wherein A is $CR_{10}$ in which $R_{10}$ is OH with an alkylating agent containing the group $R_a$ where $R_a$ is, optionally substituted alkyl to give a compound of formula (I) wherein $R_{10}$ is optionally substituted alkoxy; or

v) converting a compound of formula (I) having a reactive substituent group to a different compound of formula I.

With regard to processes (i) and (ii) the sulphonylation may be conveniently carried out in base, e.g sodium hydride, using a sulphonylating agent such as a sulphonyl chloride of formula

$R_8SO_2Cl$

wherein $R_8$ is as defined above, followed by removal of the protecting group in the case of process (i).

Process (iii) may be conveniently carried out by using an alkylating or acylating agent with an appropriate leaving group L such as a compound of formula:

$R_1$ hal

where $R_1$ is optionally substituted alkyl or alkanoyl, and hal is a halogen such as chlorine.

With regard to process (iv) the alkylation may conveniently be carried out in the presence of base, e.g. NaH, if desired in the presence of a solvent using an alkylating agent such as an alkyl halide.

Methods for converting reactive substituent groups in compounds of formula I to other substituent groups are well known to those skilled in the art. For example benzyl groups may be removed and replaced by hydrogen. Acetylamino groups may be converted to amino groups by hydrolysis.

In any of the reactions described herein reactive substituent groups or sites in the molecule may be protected prior to reaction by use of appropriate protecting groups inert to the reaction conditions and removing said protecting groups after the reaction .

In detail compounds of the invention may be prepared using conventional synthetic methods and, if required, standard separation and isolation techniques.

## FLOW DIAGRAM I

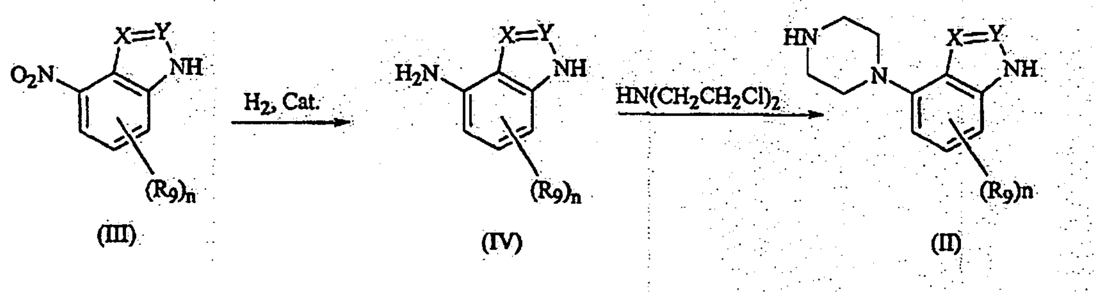


The formula II intermediate may then be converted to a compound of formula I wherein A is N, m is 2; $R_1$ is H; $R_2$, $R_3$, and $R_4$ are H; ---- represents a single bond; and the heterocyclyl group is in the 4-position, by reacting the formula II intermediate with a protecting group, G, for example di-t-butyl dicarbonate, to selectively protect the piperazine basic N atom to give the compound of formula V and sequentially reacting said formula V compound with a base such as NaH and a sulfonyl chloride, $R_8SO_2Cl$ and deprotecting it to give the desired compound of formula Ia. Reaction of said formula Ia compound with a reagent $R_1$-Hal, wherein $R_1$ is defined hereinabove for formula I and Hal is Cl, Br or I in the presence of a base gives compounds of formula Ib wherein $R_1$ is other than H. The reaction sequence is shown in flow diagram II.

## FLOW DIAGRAM II

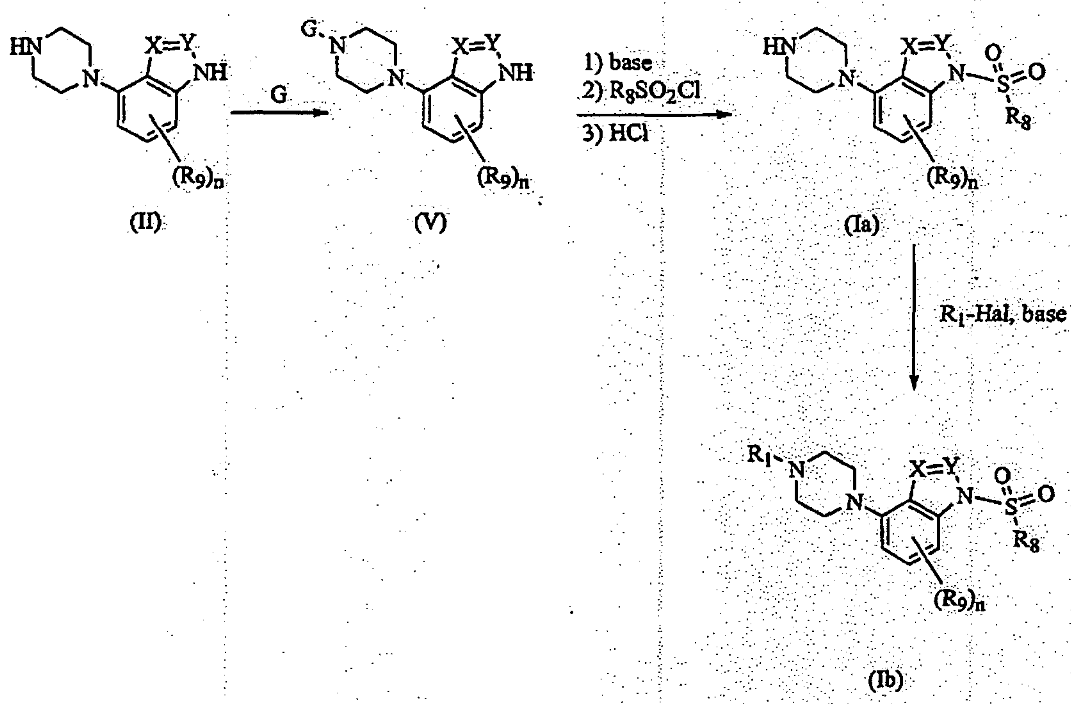


Corresponding compounds of the invention wherein A is $CR_{10}$ may be obtained, for example, by lithiating a protected compound of formula VI wherein G is benzyl, and displacing the lithium group with a cyclic ketone such as an N-protected-4-piperidone to give the hydroxy intermediate of formula VII, which may then be dehydrated and sulfonylated in the manner described hereinabove to give the protected compound of formula VIII. Catalytic hydrogenation and simultaneous deprotection of said formula VIII compound gives the desired compounds of formula I wherein ---- represents a single bond (formula Id). The reaction sequence is shown in flow diagram III.

FLOW DIAGRAM III

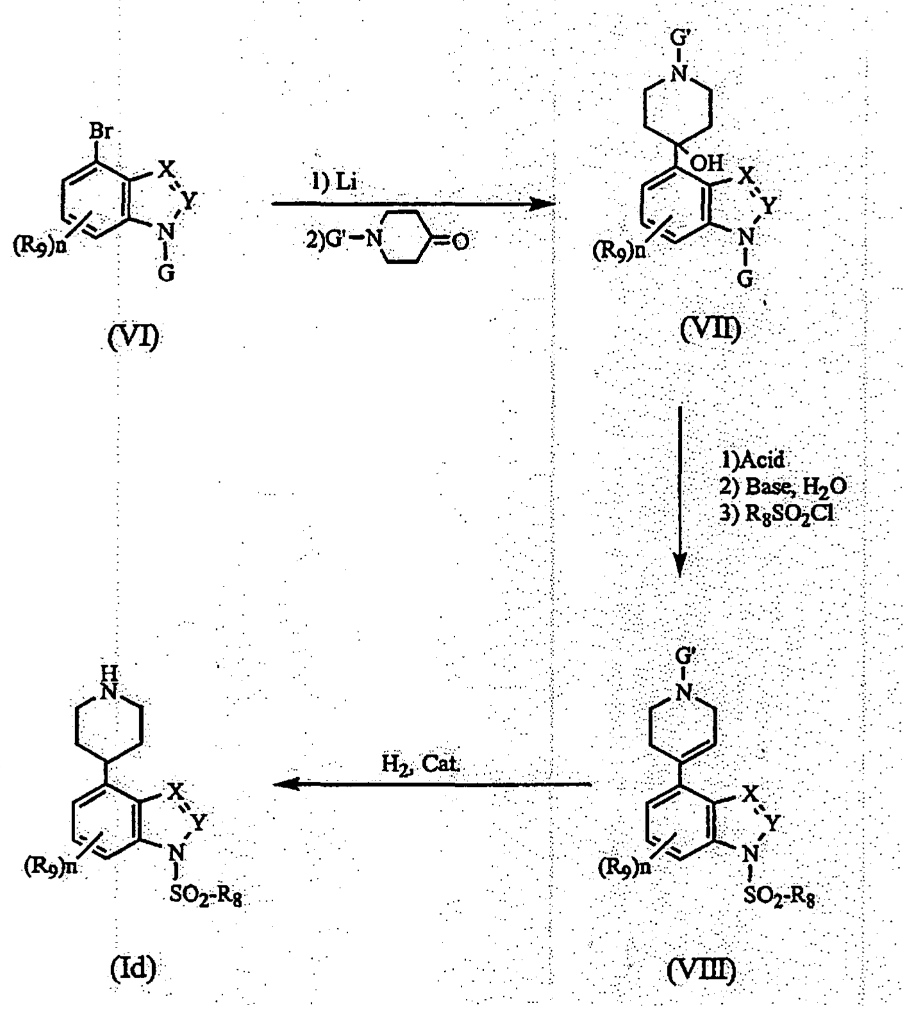


These and other literature procedures may be utilized to prepare the formula I compounds of the invention. Employing a 5-, 6- or 7- -halobenzimidazole substrate as starting material and using essentially the same procedures illustrated in flow diagrams I, II and III hereinabove enables the construction of the corresponding compounds of formula I wherein the heterocyclyl group is in the 5-, 6-, or 7-position and X is N.

Advantageously, the inventive compound of formula I may be utilized in the treatment of central nervous system disorders relating to or affected by the 5-HT6 receptor such as motor, mood, psychiatric, cognitive, neurodegenerative or the like disorders. Accordingly, the present invention provides a compound of formula I for use in the treatment of a disorder of the central nervous system (CNS) related to or affected by the 5-HT6 receptor. The compounds may be administered orally or parenterally or in any common manner known to be an effective administration of a therapeutic agent to a patient in need thereof.

The therapeutically effective amount administered in the treatment of a specific CNS disorder may vary according to the specific condition(s) being treated, the size, age and response pattern of the patient, the severity of the disorder, the judgment of the attending physician and the like. In general, effective amounts for daily oral administration may be about 0.01 to 1,000 mg/kg, preferably about 0.5 to 500 mg/kg and effective amounts for parenteral administration may be about 0.1 to 100 mg/kg, preferably about 0.5 to 50 mg/kg.

In actual practice, the compounds of the invention are administered in a solid or liquid form, either neat or in combination with one or more conventional pharmaceutical carriers or excipients. Accordingly, the present invention provides a pharmaceutical composition which comprises a pharmaceutically acceptable carrier and an effective amount of a compound of formula I as described hereinabove.

Solid carriers suitable for use in the composition of the invention include one or more substances which may also act as flavoring agents, lubricants, solubilizers, suspending agents, fillers, glidants, compression aids, binders,

tablet-disintegrating agents or encapsulating materials. In powders, the carrier may be a finely divided solid which is in admixture with a finely divided compound of formula I. In tablets, the formula I compound is mixed with a carrier having the necessary compression properties in suitable proportions and compacted in the shape and size desired. Said powders and tablets may contain up to 99% by weight of the formula I compound. Solid carriers suitable for use in the composition of the invention include calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Any pharmaceutically acceptable liquid carrier suitable for preparing solutions, suspensions, emulsions, syrups and elixirs may be employed in the composition of the invention. Compounds of formula I may be dissolved or suspended in a pharmaceutically acceptable liquid carrier such as water, an organic solvent, or a pharmaceutically acceptable oil or fat, or a mixture thereof. Said liquid composition may contain other suitable pharmaceutical additives such as solubilizers, emulsifiers, buffers, preservatives, sweeteners, flavoring agents, suspending agents, thickening agents, coloring agents, viscosity regulators, stabilizers, osmoregulators, or the like. Examples of liquid carriers suitable for oral and parenteral administration include water (particularly containing additives as above, e.g., cellulose derivatives, preferably sodium carboxymethyl cellulose solution), alcohols (including monohydric alcohols and polyhydric alcohols, e.g., glycols) or their derivatives, or oils (e.g., fractionated coconut oil and arachis oil). For parenteral administration the carrier may also be an oily ester such as ethyl oleate or isopropyl myristate.

Compositions of the invention which are sterile solutions or suspensions are suitable for intramuscular, intraperitoneal or subcutaneous injection. Sterile solutions may also be administered intravenously. Inventive compositions suitable for oral administration may be in either liquid or solid composition form.

For a more clear understanding, and in order to illustrate the invention more clearly, specific examples thereof are set forth hereinbelow. The following examples are merely illustrative and are not to be understood as limiting the scope and underlying principles of the invention in any way.

Unless otherwise stated, all parts are parts by weight. The terms HPLC and NMR designate high performance liquid chromatography and nuclear magnetic resonance, respectively.

## REFERENCE EXAMPLE 1

### Preparation of 1-(Phenylsulfonyl)-4-piperazin-1-yl-1H-indole Hydrochloride

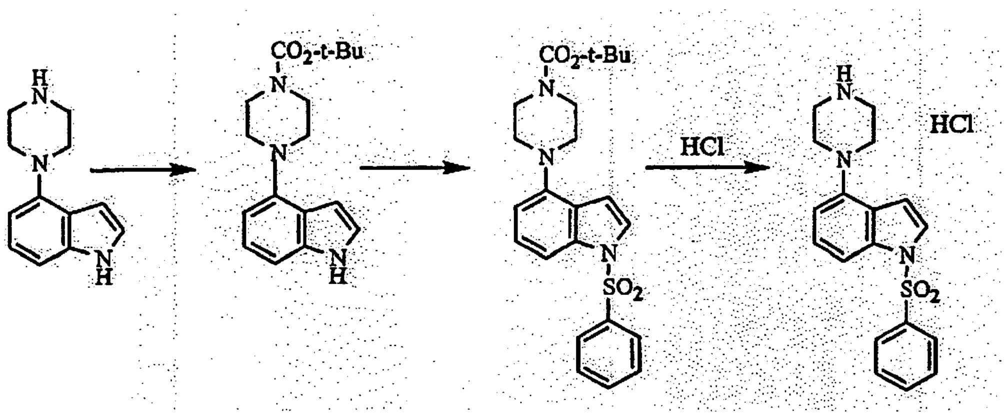


A mixture of 1H-indol-4-ylpiperazine (4.0 g, 20 mmol), di-t-butyl dicarbonate (4.8 g, 22 mmol) and NaOH (0.8 g, 20 mmol) in 40% dioxane is stirred at room temperature for 10 hours and treated with water. The reaction mixture is extracted with ethyl acetate. The extracts are combined, dried over $Na_2SO_4$ and concentrated *in vacuo* to give t-butyl 4-(1H-indol-4-yl)piperazine-1-carboxylate as a colorless solid, mp 137°C, identified by mass spectral and elemental analyses.

A portion of the t-butyl 4-(1H-indol-1-yl)-piperazine-1-carboxylate (1.05 g, 3.5 mmol) is added to a suspension of NaH (3.8 mmol) in tetrahydrofuran at 0°C under $N_2$. The resultant mixture is stirred for 0.5 hr, treated with benzenesulfonyl chloride (0.616 g, 3.5 mmol), stirred for 16 hr and treated with water. The aqueous reaction mixture is extracted with ethyl acetate. The extracts are combined, dried over $Na_2SO_4$ and concentrated *in vacuo* to give a residue. The residue is chromatographed ($SiO_2$, $CH_2Cl_2$) to give t-butyl 4-(1-phenylsulfonyl-(1H-indol-4-yl)piperazine-1-carboxylate as a light yellow solid, 1.25 g (81% yield), mp 64-65°C, identified by mass spectral and elemental analyses.

A portion of the t-butyl 4-(1-benzenesulfonyl-1H-indol-4-yl)piperazine-1-carboxylate (0.85 g) is stirred in a mixture of 4N HCl and dioxane at room temperature for 2 hrs and filtered. The filtercake is dried to give the title product

as a while solid, 0.64 g (99% yield) mp 60˚C identified by mass spectral and NMR analyses.

**REFERENCE EXAMPLES 2-13**

**Preparation of 1-Arylsulfonyl-4-Piperazin-1-yl)-1H-Indole Hydrochloride**

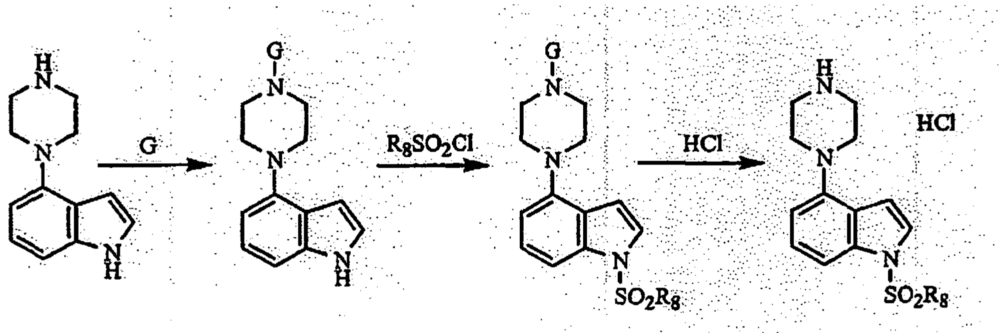


G = protecting group

Using essentially the same procedure described in Reference Example 1 and substituting the appropriate arylsulfonyl chloride, the following compounds listed in Table I are obtained and identified by HPLC and mass spectral analyses.

**TABLE I**

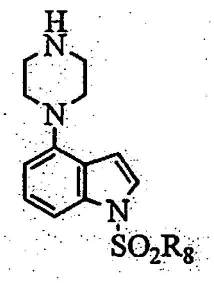

$R_8$

| REF. Ex. No. | $R_8$ | LCMS[1] Min. | M+H |
|---|---|---|---|
| 2 | o-bromophenyl | 2.58 | 422 |
| 3 | 6-chloroimidazo[2,1-b]thiazol-5-yl | 2.48 | 422 |
| 4 | 3,4-dimethoxyphenyl | 2.52 | 402 |
| 5 | 4-aminophenyl | 2.26 | 357 |
| 6 | benzo-2,1,3-thiazol-4-yl | | |
| 7 | benzofurazan-4-yl | | |
| 8 | 3-bromo-5-chlorothien-2-yl | | |
| 9 | 5-chloro-3-methylbenzo(b)thien-2-yl | | |
| 10 | Dansyl | | |
| 11 | 2,5-dichlorothien-3-yl | | |
| 12 | 3,5-dimethylisoxasol-4-yl | | |

(continued)

| REF. Ex. No. | $R_8$ | LCMS[1] Min. | M+H |
|---|---|---|---|
| 13 | 1-methylimidazol-4-yl | | |

[1] LCMS conditions: Hewlett Packard 1100 MSD; YMC ODS-AM 2.0 mm x 50 mm 5 u column at 23˚C; 3uL injection; Solvent A: 0.02% TFA/water; Solvent B:0.02% TFA/acetonitrile; Gradient: Time 0:95% A; 0.3 min: 95% A; 4.7 min: 10% A, 4.9 min: 95% A; Post time 1 min. Flow rate 1.5 mL/min; Detection: 254 nm DAD; API-ES Scanning Mode Positive 150-700; Fragmentor 70 mV.

**REFERENCE EXAMPLE 14**

**Preparation of 4-[4-(4,5-Dihydro-1H-imidazol-2-yl)-piperazin-1-yl]-1-(phenylsulfonyl)-1H-indole**

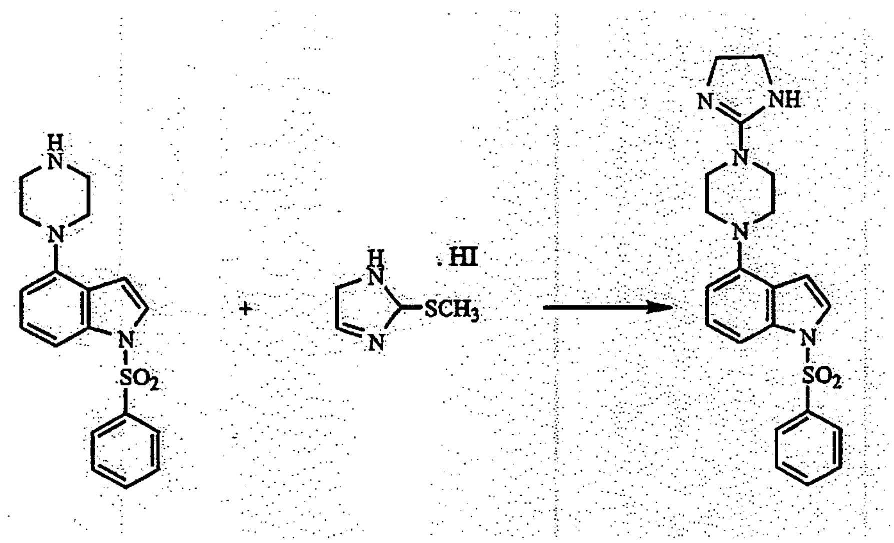

A solution of 1-(phenylsulfonyl)-4-piperazin-1-yl-1H-indole (71 mg, 0.18 mmol) in dioxane is treated with 2-methylthio-2-imidazoline hydroiodide (52.7 mg, 0.22 mmol) and N,N-diisopropylethylamine (62 μl, 0.36 mmol), heated at 50˚C for 16 hr., cooled and concentrated *in vacuo* to give a residue. The residue is purified by HPLC to give the title product, 15 mg, identified by HPLC and mass spectral analyses (2.57 min; 410 M+H) using the LCMS conditions described in Table I.

**REFERENCE EXAMPLES 15-18**

**Preparation of 4-Heterocyclyl-l-(arylsulfonyl)indole compounds**

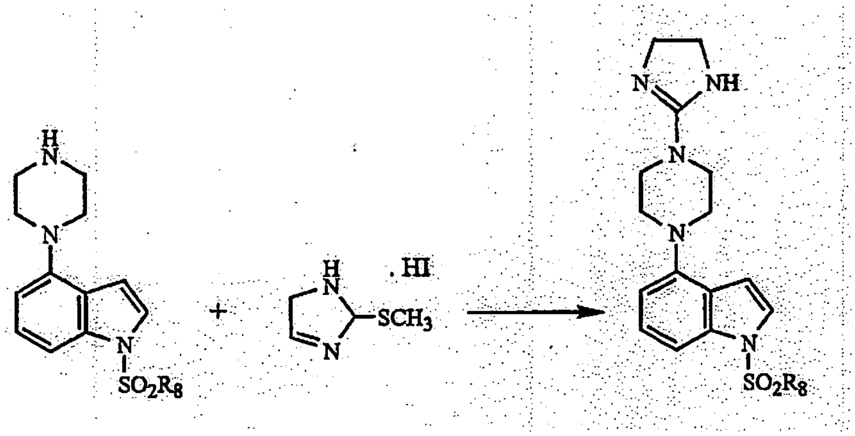

Using essentially the same procedure described in Reference Example 14 and substituting the appropriate 1-(arylsulfonyl)indole substrate, the following compounds shown in Table II are obtained and identified by HPLC and mass spectral analyses.

**TABLE II**

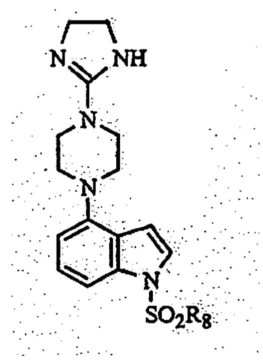

| REF. Ex. No . | $R_8$ | LCMS[1] Min. | LCMS[1] M+H |
|---|---|---|---|
| 15 | 2-bromophenyl | 2.79 | 490 |
| 16 | 6-chloroimidazo[2,1-b]thiazol-5-yl | 2.68 | 490 |
| 17 | 3,4-dimethoxyphenyl | 2.64 | 470 |
| 18 | 4-aminophenyl | 2.46 | 425 |

[1] LCMS conditions: same as for Table I

**REFERENCE EXAMPLE 19**

**Preparation of 4-(4-Benzylpiperazin-1-yl)-1-(phenyl-sulfonyl)-1H-indole**

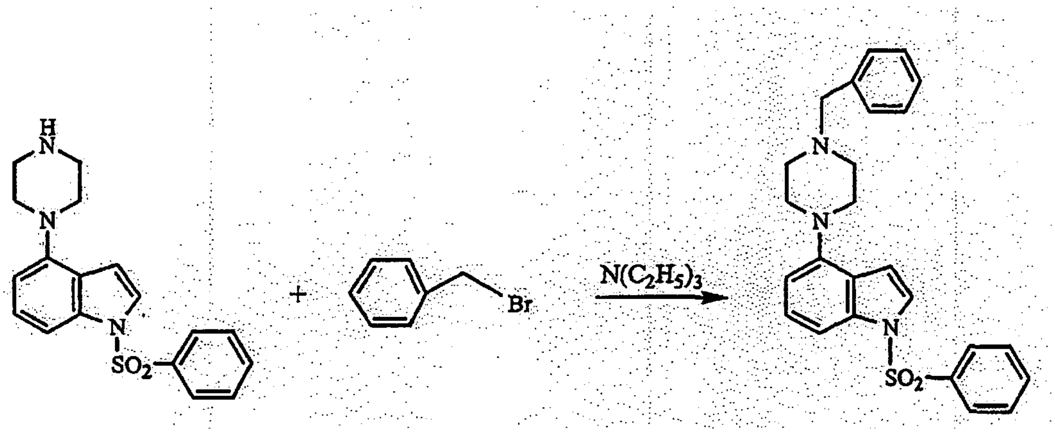


A solution of 1-(phenylsulfonyl)-4-piperazin-1-yl-1H-indole (71 mg, 0.18 mmol) in tetrahydrofuran is treated sequentially with benzyl bromide (21 μl) and triethyl-amine (75 μl), shaken at room temperature for 16 hr and concentrated *in vacuo* to give a residue. The residue is purified by RP-HPLC to give the title product, 37 mg, identfied by HPLC and mass spectral analyses (2.81 min; 432 M+H) using the LCMS conditions described in Table I.

**REFERENCE EXAMPLES 20-53**

**Preparation of 4-Heteroaryl-1-arylsulfonylindole compounds**

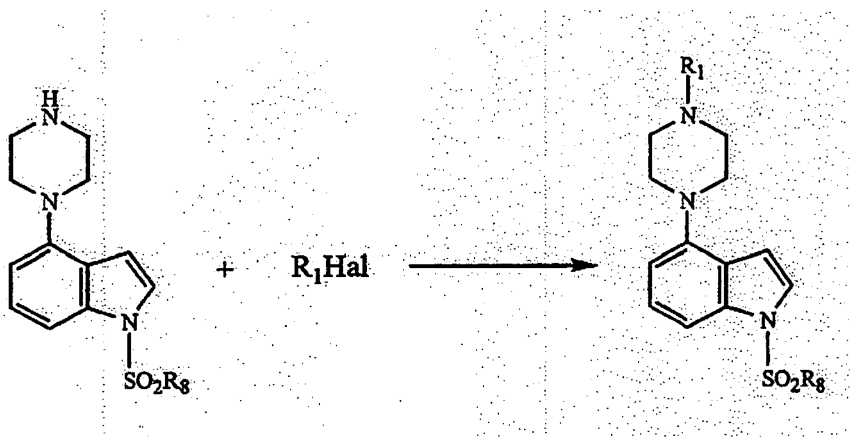

Using essentially the same procedure described in Reference Example 19 and employing the appropriate 4-(piperazin-1-yl)-1-(arylsulfonyl)indole substrate and a suitable aryl, alkyl or acyl halide, the following compounds shown in Table III are obtained and identified by HPLC and mass spectral analyses.

**TABLE III**

| |
|---|
| 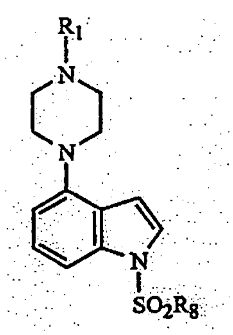 |

(continued)

| REF. Ex. No. | $R_1$ | $R_8$ | LCMS[1] Min. | LCMS[1] M+H |
|---|---|---|---|---|
| 20 | 2-chloro-5- thienylmethyl | phenyl | 3.07 | 472 |
| 21 | 3-nitrobenzyl | phenyl | 2.95 | 477 |
| 22 | acetyl | phenyl | 3.18 | 384 |
| 23 | benzyl | 2-bromophenyl | 2.99 | 512 |
| 24 | 2-chloro-5- thienylmethyl | 2-bromophenyl | 3.08 | 550 |
| 25 | 3-nitrobenzyl | 2-bromophenyl | 3.08 | 550 |
| 26 | acetyl | 2-bromophenyl | 2.97 | 557 |
| 27 | benzyl | 6-choroimidazol[2,1- b]thiazol-5-yl | 2.91 | 512 |
| 28 | 2-chloro-5- thienylmethyl | 6-choroimidazol[2,1- b]thiazol-5-yl | 3.00 | 553 |
| 29 | 3-nitrobenzyl | 6-choroimidazol[2,1- b]thiazol-5-yl | 2.87 | 557 |
| 30 | acetyl | 6-choroimidazol[2,1- b]thiazol-5-yl | 3.23 | 464 |
| 31 | benzyl | 3,4-dimethoxyphenyl | 2.76 | 492 |
| 32 | 2-chloro-5- thienylmethyl | 3,4-dimethoxyphenyl | 2.90 | 532 |
| 33 | 3-nitrobenzyl | 3,4-dimethoxyphenyl | 2.82 | 537 |
| 34 | acetyl | 3,4-dimethoxyphenyl | 3.10 | 442 |
| 35 | benzyl | 4-aminophenyl | 2.64 | 447 |
| 36 | methyl | 4-aminophenyl | 2.28 | 371 |
| 37 | 2-chloro-5- thienylmethyl | 4-aminophenyl | 2.82 | 487 |
| 38 | 3-nitrobenzyl | 4-aminophenyl | 2.72 | 492 |
| 39 | 3-methoxybenzyl | phenyl | 2.88 | 462 |
| 40 | 4-pyridylmethyl | phenyl | 2.40 | 433 |
| 41 | 3-pyridylmethyl | phenyl | 2.42 | 433 |
| 42 | 3-methoxybenzyl | 2-bromophenyl | 2.99 | 542 |
| 43 | 4-pyridylmethyl | 2-bromophenyl | 2.51 | 513 |
| 44 | 3-pyridylmethyl | 2-bromophenyl | 2.52 | 513 |
| 45 | 3-methoxybenzyl | 6-chloroimidazo[2,1- b] thiazol-5-yl | 2.93 | 542 |
| 46 | 4-pyridylmethyl | 6-chloroimidazo[2,1- b] thiazol-5-yl | 2.48 | 513 |
| 47 | 3-pyridylmethyl | 6-chloroimidazo[2,1- b]thiazol-5-yl | 2.48 | 513 |
| 48 | 3-methoxybenzyl | 3,4-dimethoxyphenyl | 2.82 | 522 |
| 49 | 4-pyridylmethyl | 3,4-dimethoxyphenyl | 2.47 | 493 |
| 50 | 3-pyridylmethyl | 3,4-dimethoxyphenyl | 2.45 | 493 |
| 51 | 3-methoxybenzyl | 4-aminophenyl | 2.75 | 477 |
| 52 | 4-pyridylmethyl | 4-aminophenyl | 2.24 | 448 |
| 53 | 3-pyridylmethyl | 4-aminophenyl | 2.26 | 448 |

[1]LCMS conditions are the same as that for Table I

**EXAMPLE 54**

**Preparation of 4-(Homopiperazin-1-yl)-1-(phenylsulfonyl)-benzimidazole hydrochloride**

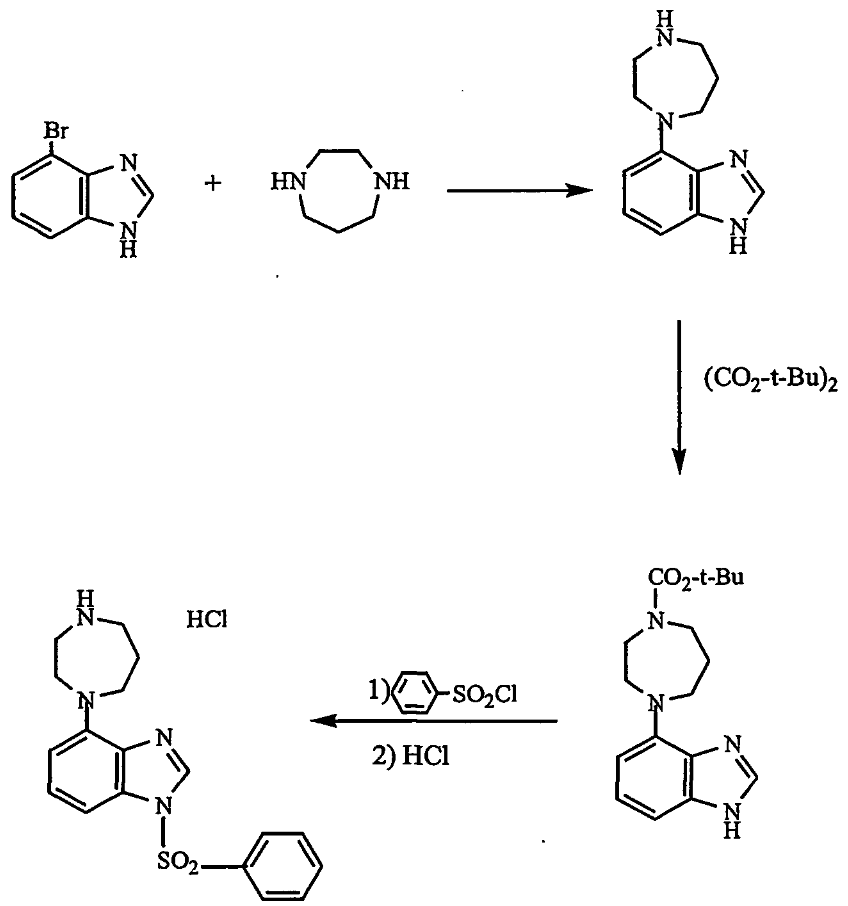


A suspension of 4-bromobenzimidazole (42 mmol), homopiperazine (256 mmol) and NaOt-Bu (59 mmol) in dry o-xylene, under $N_2$, is treated with a catalytic amount of Pd $(OCOCH_3)_2 \cdot P(t\text{-}Bu)_3$ (P/Pd = 4), heated at 120°C for 3 hr, cooled to room temperature and diluted with water. The aqueous mixture is extracted with ethyl acetate. The extracts are combined, dried over $MgSO_4$ and concentrated *in vacuo* to give a residue. The residue is purified by flash chromatography to give 4-(homopiperazin-l-yl)benzimidazole.

A mixture of 4-(homopiperazin-1-yl)benzimidazole (4.3 g, 20 mmol), di-t-butyl dicarbonate (4.8 g, 22 mmol) and NaOH (0.8 g, 20 mmol) in 40% aqueous dioxane is stirred at room temperature for 10 hrs and diluted with water. The aqueous mixture is extracted with ethyl acetate. The extracts are combined, dried over $NaSO_4$ and concentrated *in vacuo* to give t-butyl 4-(benzimidazol-4-yl)homopiperazine-1-carboxylate.

A suspension of NaH (3.8 mmol) in tetrahydrofuran at 0°C, under $N_2$, is treated with t-butyl 4-(benzimidazol-4-yl)- homopiperazine-1-carboxylate (1.1g, 3.5 mmol), stirred for 0.5 hr, treated with benzenesulfonyl chloride (0.616 g, 3.5 mmol), stirred for 16 hours at room temperature and diluted with water. The aqueous mixture is extracted with ethyl acetate. The extracts are combined, dried over $Na_2SO_4$ and concentrated *in vacuo* to give a residue. The residue is purified by flash chromatography to give t-butyl 4-(1-phenylsulfonyl)-benzimidazol-4-yl)homopiperazin-1-carboxylate.

A mixture of the thus-obtained carboxylate in 4N HCl and dioxane is stirred at room temperature for 2 hrs and filtered. The filtercake is washed with ethyl acetate and dried *in vacuo* to afford the title product.

## REFERENCE EXAMPLE 56

### Preparation of 4-(4-Benzylpiperazin-1-yl)-1H-indazole

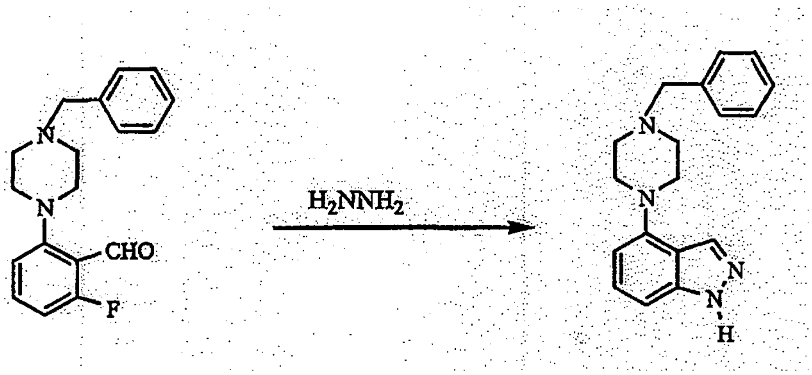


A stirred solution of 4-benzyl-1-(3-fluoro-2-carboxyphenyl)-piperazine (5.96 g, 20.0 mmol) in dimethylsulfoxide (10 mL) and hydrazine (10 mL) is heated at 95˚C under nitrogen for 4 days. The cooled reaction is diluted with ether and washed with a mixture of water and saturated aqueous sodium bicarbonate. The organic layer is further washed sequentially with water and brine dried over $MgSO_4$ and concentrated *in vacuo* to give a residue. The residue is chromatographed using ethyl acetate as the eluant. The resulting oil is reconcentrated from ether to give a white foam which is stirred under hexanes/ether overnight. The resulting white powder is isolated by suction filtration and washed with hexane to give the title compound 3.11 g, (53% yield), identified by HNMR.

**REFERENCE EXAMPLE 57**

**Preparation of 4-(4-Benzylpiperazin-1-yl)-1-(phenylsulfonyl)-1H-indazole hydrochloride**

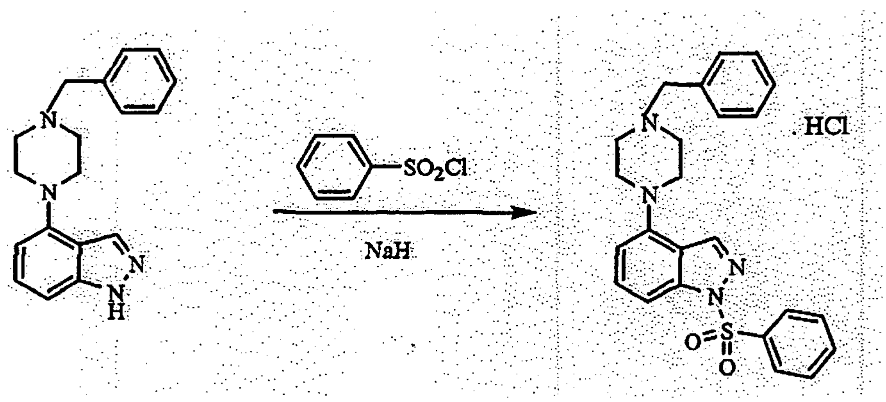


A solution of 4-(4-benzylpiperazin-1-yl)-1H-indazole (2.34 g, 8.00 mmol) in dry dimethyl formamide is treated with 0.48 g unwashed 60% NaH in mineral oil (12.0 mmol of NaH). After stirring under nitrogen for 15 min, the reaction is treated with benzenesulfonylchloride (1.53 mL, 12.0 mmol), stirred for 24 hr at ambient temperature, treated with saturated aqueous $NaHCO_3$ and water and extracted with ether. The organic layer is washed sequentially with water and brine, dried over $MgSO_4$ and concentrated *in vacuo* to give a residue. The residue is purified by flash chromatography on silica gel using 1:1 ethyl acetate:hexanes as eluant to afford the free amine of the title compound as an oil (3.14 g, 91%). A portion of this oil (432 mg, 1.0 mmol) is dissolved in ether and treated with 1.0M HCl in ether (1.1 mL, 1.1 mmol). The resulting solid is filtered, washed with ether, and dried under vacuum to provide the title compound as a light tan solid, mp 208-209˚C, identified by HNMR and mass spectral analyses.

**REFERENCE EXAMPLE 58**

**Preparation of 1-(Phenylsulfonyl)-4-(1-piperazinyl)-1H-indazole hydrochloride**

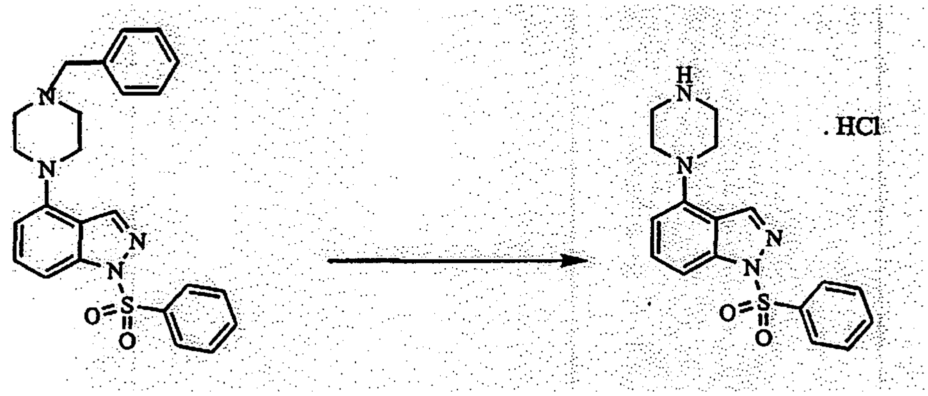

A solution of 1-phenylsulfonyl-4-(4-benzylpiperazin-1-yl)-1H-indazole (433 mg, 1.0 mmol) in 1,2-dichloroethane is treated with 1-chloroethyl chloroformate (0.27 mL, 2.5 mmol) heated at reflux temperature for 2 hr, and concentrated *in vacuo.* The resultant residue is heated at reflux temperature in methanol for 1.5 hr, cooled, concentrated *in vacuo* and reconcentrated from ether. The resulting tan solid is triturated with ether and crystallized from hot ethanol to give the title compound as a tan solid 237 mg (63% yield), mp 203-205 ˚C, identified by HNMR and mass spectral analyses.

**REFERENCE EXAMPLE 59**

**Preparation of 4-[4-(2-phenylethyl)piperazin-1-yl]-1-(phenylsulfonyl)-1H-indazole hydrochloride**

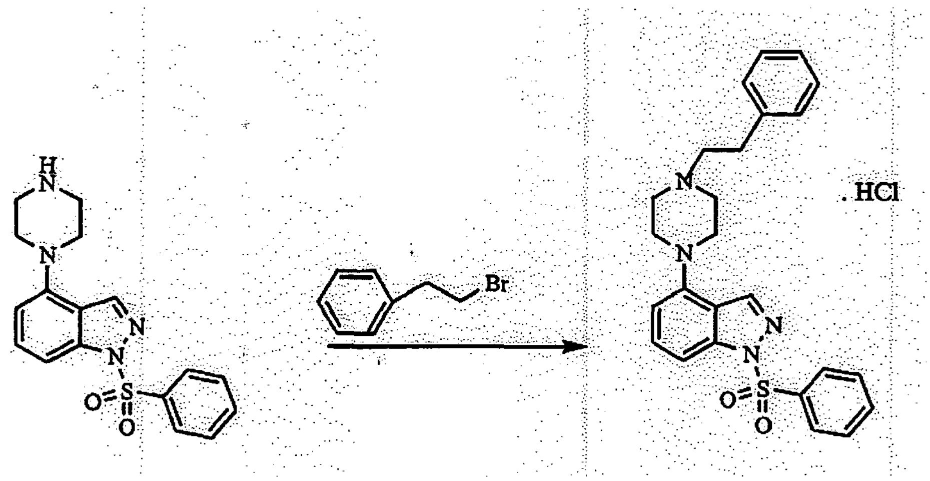

A mixture of 1-phenylsulfonyl-4-piperazin-1-yl-1H-indazole (190 mg, 0.50 mmol) and $K_2CO_3$ (138 mg, 1.0 mmol) in dry acetonitrile is treated with phenethylbromide (0.55 mL, 2.0 mmol), heated at reflux temperature under nitrogen for 8.5 h, treated with water and extracted with methylene chloride. The combined extracts are dried over $MgSO_4$ and chromatographed on an SCX column (Varian SCX Mega Bond Elut, 5 g) eluting with ethyl acetate to remove non-basic organic material and then with 1:99 triethylamine:ethyl acetate to afford, after concentration, the free amine of the title compound as a slightly yellow oil (198 mg, 89%). The oil is dissolved in ether with a small amount of ethanol to aid solubility and treated with 1.0M HCl in ether. The solution is concentrated *in vacuo* and the resulting tan solid is treated with ether and suction filtered to afford the title compound as a light tan solid 209 mg, (87% yield), mp 230-232 ˚C (dec), identified by NMR and mass spectral analyses.

**REFERENCE EXAMPLES 60-72**

**Preparation of 4-Heteroaryl-l-arylsulfonylindazole compounds**

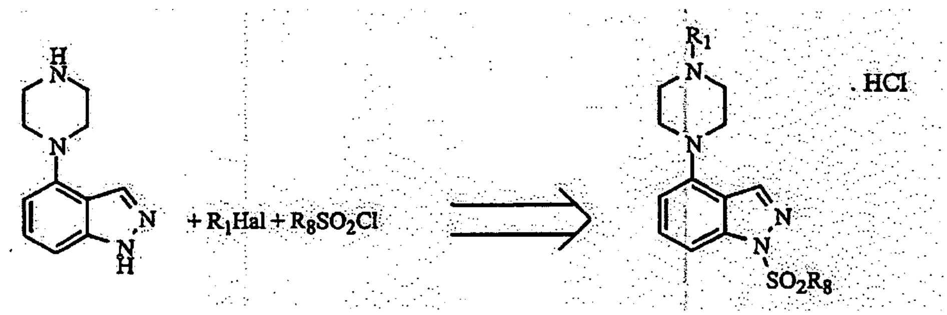

Using essentially the same procedures described in Reference Examples 56-59 and employing the appropriate indazole substrate and suitable aryl, alkyl or acyl halide or arylsulfonyl chloride, the following compounds shown in Table IV are obtained and identified by NMR and mass spectral analyses.

**TABLE IV**

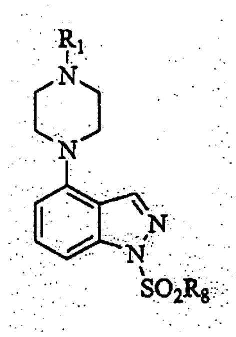

| REF. Ex. No. | $R_1$ | $R_8$ | mp ˚C | M+H |
|---|---|---|---|---|
| 60 | 2(p-fluorophenoxy)ethyl- | phenyl | 184-186 | 481 |
| 61 | p-fluorophenyl-CO-$(CH_2)_3$- | phenyl | -- | 507 |
| 62 | phenyl-CO-$CH_2$- | phenyl | 202-205 | 461 |
| 63 | 3-phenylpropyl- | phenyl | 188-190 | 461 |
| 64 | n-propyl- | phenyl | 258-260 | 385 |
| 65 | benzyl | phenyl-CH=CH- | 233-235 | 459 |
| 66 | benzyl | p-fluorophenyl | 240-241 | 451 |
| 67 | benzyl | p-chlorophenyl | 238-239 | 467 |
| 68 | benzyl | naphthyl | 147-149 | 483 |
| 69 | benzyl | p-methoxyphenyl | 206-209 | 463 |
| 70 | benzyl | p-(trifluoro- methoxy)phenyl | 229-231 | 517 |
| 71 | benzyl | 2-(4,5- dichloro-thienyl)- | 235-237 | 507 |
| 72 | benzyl | p-tolyl | 215-217 | 447 |

**REFERENCE EXAMPLE 73**

**Preparation of 1-(4-Aminophenylsulfonyl)-5-piperazin-1-yl-1H-indole hydrochloride**

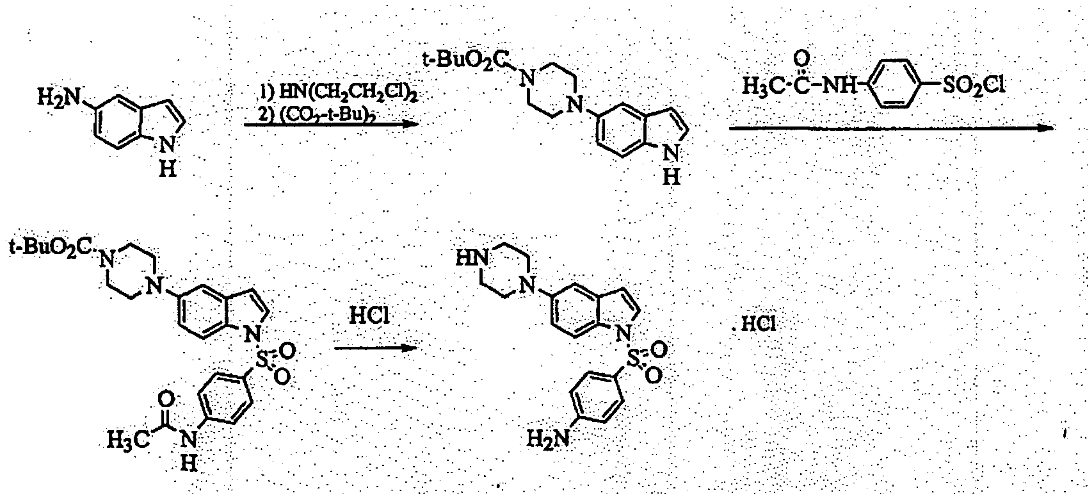


A solution of 5-aminoindole (6.23 g, 47 mmol), bis(2-chloroethyl)amine hydrochloride (16.8 g, 96 mmol) and triethylamine (19 mL, 141 mmol) in butanol is heated at 100°C for 8 hours, cooled to room temperature and concentrated *in vacuo* to give 9.46 g of 5-piperazin-1-yl-1H-indole.

A solution of said indole in acetone and water is treated with di-tert-butyl dicarbonate (11.3 g, 47 mmol) and potassium carbonate (13 g, 96 mmol). The mixture is stirred at room temperature overnight, the acetone evaporated and the remaining aqueous phase extracted with ethyl acetate. The extracts are dried over $MgSO_4$ and concentrated *in vacuo* to give a residue. The residue is purified by flash chromatography to give 4-(1H-indol-5-yl)-piperazine-1-carboxylic acid *tert*-butyl ester.

A solution of said ester (60 mg, 0.2 mmol) in tetrahydrofuran is treated with sodium hydride (30 mg, 0.5 mmol) followed by N-acetylsulfanilyl chloride (25 uL, 0.2 mmol), shaken at room temperature for 16 hours and concentrated *in vacuo* to give 4-[1-(4-acetylaminophenylsulfonyl)-1H-indol-5-yl]-piperazine-1-carboxylic acid tert-butyl ester.

The thus-obtained ester is dissolved in methanol, treated with concentrated hydrochloric acid (100 uL), shaken at 60°C for 2 hours and concentrated *in vacuo* to give a residue. The residue is purified by HPLC to give the title product, 15 mg, identified by HPLC and mass spectral analyses (r.t. 2.37 min., M+H 357).

**EXAMPLES 74-75, 77-82, 84-85, 87-94, 96, 98-99 and 101-102 and REFERENCE EXAMPLES 76, 83, 86, 95, 97 and 100.**

**Preparation of Piperazinyl-1-arylsulfonylbenzimidazole and indole compounds**

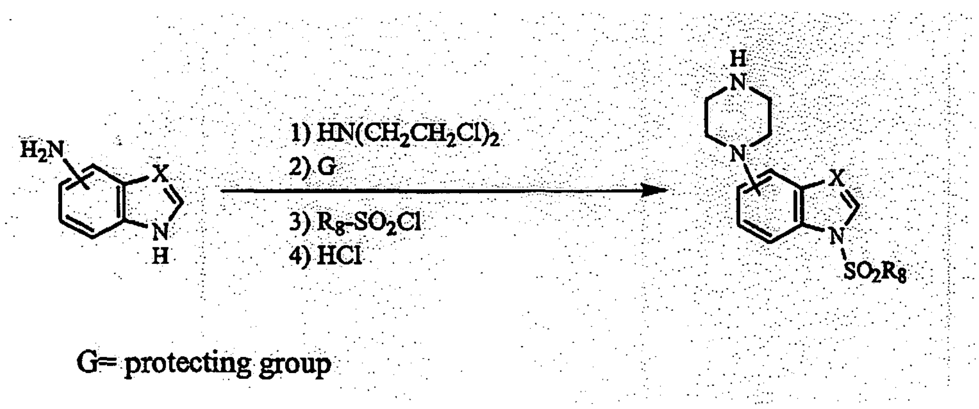


Using essentially the same procedures described in Reference Example 73 and employing the appropriate aminoindole or aminobenzimidazole substrate and suitable arylsulfonylchloride reagents, the following compounds shown in Table V are obtained and identified by HPLC and mass spectral analyses.

**TABLE V**

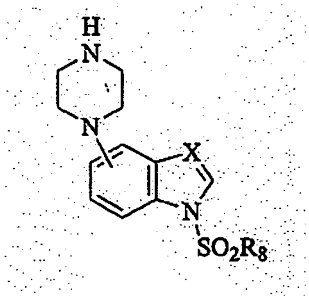

| Ex. No. | Piperazinyl Ring Position | X | $R_8$ | LCMS[1] Min. | M+H |
|---|---|---|---|---|---|
| 74 | 5 | N | phenyl | 1.98 | 343 |
| 75 | 6 | N | phenyl | 1.96 | 343 |
| REF. 76 | 5 | CH | benzo-2,1,3-thiadiazol-4-yl | 2.56 | 400 |
| 77 | 6 | N | benzo-2,1,3-thiadiazol-4-yl | 2.01 | 401 |
| 78 | 6 | N | 2-bromophenyl | 2.21 | 423 |
| 79 | 5 | N | p-bromophenyl | 2.39 | 423 |
| 80 | 6 | N | p-bromophenyl | 2.34 | 423 |
| 81 | 5 | N | 5-bromothien-2-yl | 2.33 | 429 |
| 82 | 6 | N | 5-bromothien-2-yl | 2.25 | 429 |
| REF. 83 | 5 | CH | p-(n-butoxy)phenyl | 3.23 | 414 |
| 84 | 5 | N | p-(n-butoxy)phenyl | 2.79 | 415 |
| 85 | 6 | N | p-(n-butoxy)phenyl | 2.73 | 415 |
| REF. 86 | 5 | CH | 5-chloro-1,3-dimethyl- pyrazol-4-yl | 2.49 | 395 |
| 87 | 5 | N | 5-chloro-1,3-dimethyl- pyrazol-4-yl | 1.88 | 396 |
| 88 | 5 | N | 5-chloro-3-methylbenzo- [b] thien-2-yl | 2.88 | 448 |
| 89 | 6 | N | 5-chloro-3-methylbenzo- [b]thien-2-yl | 3.10 | 448 |
| 90 | 5 | N | 2,3-dichlorothien-5-yl | 2.59 | 418 |
| 91 | 6 | N | 2,3,-dichlorothien-5-yl | 2.77 | 418 |
| 92 | 5 | N | p-fluorophenyl | 2.08 | 361 |
| 93 | 6 | N | p-fluorophenyl | 2.40 | 361 |
| 94 | 5 | N | p-methoxyphenyl | 2.11 | 373 |
| REF.95 | 5 | CH | 2-naphthyl | 2.92 | 392 |
| 96 | 6 | N | 2-naphthyl | 2.43 | 393 |
| REF.97 | 5 | CH | p-(trifluoromethoxy)phenyl | 2.97 | 426 |
| 98 | 5 | N | p-(trifluoromethoxy)phenyl | 2.57 | 427 |
| 99 | 6 | N | p-(trifluoromethoxy)phenyl | 2.54 | 427 |
| REF.100 | 5 | CH | p-iodophenyl | 2.92 | 468 |
| 101 | 5 | N | p-iodophenyl | 2.48 | 469 |
| 102 | 6 | N | p-iodophenyl | 2.67 | 469 |

**EXAMPLE 103**

**Comparative Evaluation of 5-HT6 Binding Affinity of Test Compounds**

The affinity of test compounds for the serotonin 5-HT6 receptor is evaluated in the following manner. Cultured Hela cells expressing human cloned 5-HT6 receptors are harvested and centrifuged at low .speed (1,000 x g) for 10.0 min to remove the culture media. The harvested cells are suspended in half volume of fresh physiological phosphate buffered saline solution and recentrifuged at the same speed. This operation is repeated. The collected cells are then

homogenized in ten volumes of 50 mM Tris.HCl (pH 7.4) and 0.5 mM EDTA. The homogenate is centrifuged at 40,000 x g for 30.0 min and the precipitate is collected. The obtained pellet is resuspended in 10 volumes of Tris.HCl buffer and recentrifuged at the same speed. The final pellet is suspended in a small volume of Tris.HCl buffer and the tissue protein content is determined in aliquots of 10-25 μl volumes. Bovine Serum Albumin is used as the standard in the protein determination according to the method described in Lowry et al., J. Biol. Chem., 193:265 (1951). The volume of the suspended cell membranes is adjusted to give a tissue protein concentration of 1.0 mg/ml of suspension. The prepared membrane suspension (10 times concentrated) is aliquoted in 1.0 ml volumes and stored at -70° C until used in subsequent binding experiments.

Binding experiments are performed in a 96 well microtiter plate format, in a total volume of 200 μl. To each well is added the following mixture: 80.0 μl of incubation buffer made in 50 mM Tris.HCl buffer (pH 7.4) containing 10.0 mM $MgCl_2$ and 0.5 mM EDTA and 20 μl of [$^3$H]-LSD (S.A., 86.0 Ci/mmol, available from Amersham Life Science), 3.0 nM. The dissociation constant, $K_D$ of the [$^3$H]LSD at the human serotonin 5-HT6 receptor is 2.9 nM, as determined by saturation binding with increasing concentrations of [$^3$H]LSD. The reaction is initiated by the final addition of 100.0 μl of tissue suspension. Nonspecific binding is measured in the presence of 10.0 μM methiothepin. The test compounds are added in 20.0 μl volume.

The reaction is allowed to proceed in the dark for 120 min at room temperature, at which time, the bound ligand-receptor complex is filtered off on a 96 well unifilter with a Packard Filtermate® 196 Harvester. The bound complex caught on the filter disk is allowed to air dry and the radioactivity is measured in a Packard TopCount ® equipped with six photomultiplier detectors, after the addition of 40.Oμl Microscint®-20 scintillant to each shallow well. The unifilter plate is heat-sealed and counted in a PackardTopCount® with a tritium efficiency of 31.0%.

Specific binding to the 5-HT6 receptor is defined as the total radioactivity bound less the amount bound in the presence of 10.0μM unlabeled methiothepin. Binding in the presence of varying concentrations of test compound is expressed as a percentage of specific binding in the absence of test compound. The results are plotted as log % bound versus log concentration of test compound. Nonlinear regression analysis of data points with a computer assisted program Prism® yielded both the $IC_{50}$ and the Ki values of test compounds with 95% confidence limits. A linear regression line of data points is plotted, from which the $IC_{50}$ value is determined and the $K_i$ value is determined based upon the following equation:

$$K_i = IC_{50} / (1 + L/K_D)$$

where L is the concentration of the radioactive ligand used and $K_D$ is the dissociation constant of the ligand for the receptor, both expressed in nM.

Using this assay, the following Ki values are determined and compared to those values obtained by representative compounds known to demonstrate binding to the 5-HT6 receptor. The data are shown in Table VI, below.

**TABLE VI**

| Test Compound (REF. Ex. No. or Ex. No.) | 5-HT6 binding Ki (nM) |
|---|---|
| 1 | 1.0 |
| 2 | 2.0 |
| 3 | 1.0 |
| 4 | 15.0 |
| 5 | 1.0 |
| 14 | 24.0 |
| 18 | 6.0 |
| 27 | 56.0 |
| 30 | 220.0 |
| 33 | 45.0 |
| 35 | 15.0 |
| 36 | 3.0 |
| 37 | 59.0 |
| 38 | 5.0 |
| 40 | 4.0 |
| 41 | 7.0 |

(continued)

| Test Compound (REF. Ex. No. or Ex. No.) | 5-HT6 binding Ki (nM) |
|---|---|
| 42 | 4.0 |
| 43 | 7.0 |
| 44 | 1.0 |
| 46 | 5.0 |
| 47 | 6.0 |
| 48 | 14.0 |
| 49 | 10.0 |
| 50 | 17.0 |
| 51 | 7.0 |
| 52 | 25.0 |
| 53 | 4.0 |
| 57 | 14 |
| 58 | 0.3 |
| 59 | 1.0 |
| 60 | 306 |
| 61 | 3.0 |
| 62 | 12 |
| 63 | 6.0 |
| 64 | 2.0 |
| 65 | 172 |
| 66 | 84 |
| 67 | 87 |
| 68 | 14 |
| 69 | 116 |
| 70 | 251 |
| 71 | 81 |
| 72 | 56 |
| 73 | 34 |
| 79 | 19 |
| 81 | 44 |
| 83 | 38 |
| 86 | 44 |
| 89 | 24 |
| 90 | 30 |
| 91 | 6 |
| 96 | 37 |
| 101 | 18 |
| **Comparative Examples** | **5-HT6 binding Ki** |
| Clozapine | 6.0 |
| Loxapine | 41.4 |
| Bromocriptine | 23.0 |
| Methiothepin | 8.3 |
| Mianserin | 44.2 |
| Olanzepine | 19.5 |

As can be seen from the results set forth above, the compounds of the present invention have a high degree of affinity for the serotonin 5-HT6 receptor sub-type. Although two of the comparison compounds (clozapine and methiothepin) have similar 5-HT6 receptor affinity, they do not have the selectivity of the compounds of the present invention. The examples disclosed above demonstrate up to 50-fold selectivity for the 5-HT6 receptor when compared to their affinity at the 5-HT7 receptor.

## Claims

**1.** A compound of formula (I)

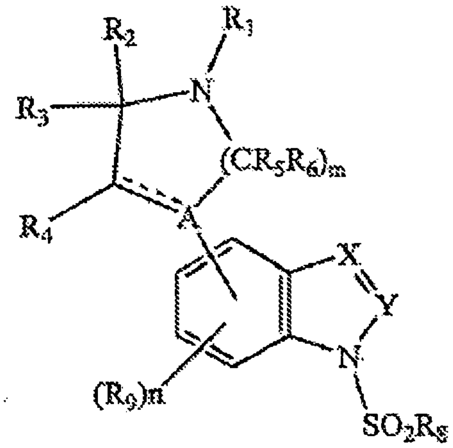

(I)

wherein
A is C, $CR_{10}$ or N;
X is N;
Y is $CR_7$;
$R_1$ is H, $C_1$-$C_6$alkylcarbonyl, $C_1$-$C_6$alkoxycarbonyl or a $C_1$-$C_6$alkyl, $C_2$-$C_6$alkenyl, $C_2$-$C_6$alkynyl or cycloheteroalkyl group each optionally substituted;
$R_2$, $R_3$, $R_4$, $R_5$ and $R_6$ are each independently H, halogen, OH or an optionally substituted $C_1$-$C_6$alkyl group;
$R_7$ is H, halogen or a $C_1$-$C_6$alkyl, aryl, heteroaryl or $C_1$-$C_6$alkoxy group each optionally substituted;
$R_8$ is a $C_1$-$C_6$alkyl, aryl or heteroaryl group each optionally substituted;
$R_9$ is H, halogen or a $C_1$-$C_6$alkyl, $C_1$-$C_6$alkoxy, $C_2$-$C_6$alkenyl, aryl or heteroaryl group each optionally substituted;
$R_{10}$ is H, OH or an optionally substituted $C_1$-$C_6$alkoxy group;
m is an integer of 1, 2 or 3;
n is 0 or an integer of 1, 2 or 3; and
------ represents a single bond or a double bond; or
a pharmaceutically acceptable salt thereof.

**2.** The compound according to claim I wherein A is N and m is 2.

**3.** The compound according to claim 1 or claim 2 wherein $R_8$ is an optionally substituted phenyl group.

**4.** The compound according to any one of claims 1 to 3 wherein $R_2$, $R_3$, $R_4$, $R_5$ and $R_6$ are H.

**5.** The compound according to any one of claims 1 to 4 wherein $R_1$ is H or a $C_1$-$C_6$alkyl or cycloheteroalkyl group each optionally substituted.

**6.** A compound according to any one of claims 1 to 5 for use in the treatment of a disorder of the central nervous system related to or affected by the 5-HT6 receptor.

**7.** The compound for use according to claim 6 wherein said disorder is a motor disorder, anxiety disorder or cognitive disorder.

**8.** The compound for use according to claim 6 wherein said disorder is schizophrenia or depression.

**9.** The compound for use according to claim 6 wherein said cognitive disorder is a neurodegenerative disorder.

**10.** The compound for use according to claim 9 wherein said neurodegenerative disorder is Alzheimer's disease or Parkinson's disease.

**11.** A pharmaceutical composition which comprises a pharmaceutically acceptable carrier and a compound of formula I or a pharmaceutically acceptable salt thereof as claimed in any one of claims 1 to 5.

**12.** A method for the preparation of a compound of formula (I):

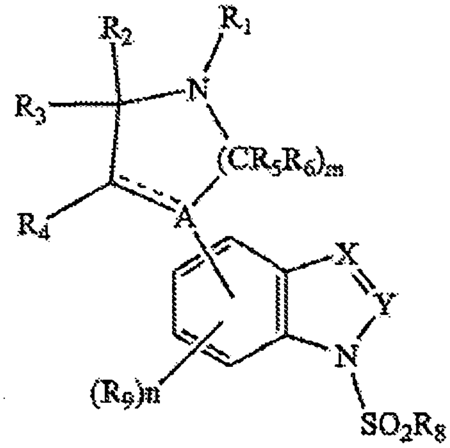

(I)

wherein
A is C, $CR_{10}$ or N;
X is N;
Y is $CR_7$;
$R_1$ is ($C_1$-$C_6$alkyl)carbonyl, ($C_1$-$C_6$alkoxy)carbonyl or a $C_1$-$C_6$alkyl, $C_2$-$C_6$alkenyl, $C_2$-$C_6$alkynyl or cycloheteroalkyl group each optionally substituted;
$R_2$, $R_3$, $R_4$, $R_5$ and $R_6$ are each independently H, halogen, OH or an optionally substituted $C_1$-$C_6$alkyl group;
$R_7$ is H, halogen or a $C_1$-$C_6$alkyl, aryl, heteroaryl or $C_1$-$C_6$alkoxy group each optionally substituted;
$R_8$ is a $C_1$-$C_6$alkyl, aryl or heteroaryl group each optionally substituted;
$R_9$ is H, halogen or a $C_1$-$C_6$alkyl, $C_1$-$C_6$alkoxy, $C_2$-$C_6$alkenyl, aryl or heteroaryl group each optionally substituted;
$R_{10}$ is H, OH or an optionally substituted $C_1$-$C_6$alkoxy group;
m is an integer of 1, 2 or 3;
n is 0 or an integer of 1, 2 or 3; and
------ represents a single bond or a double bond;
said method which comprises one of the following:

i) reacting a compound of formula:

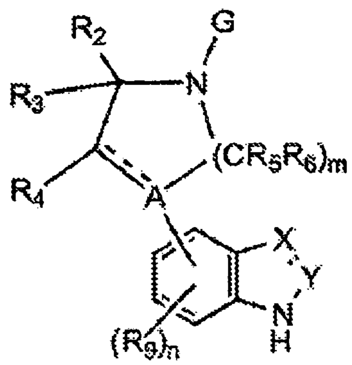

wherein the dotted line, n, m, $R_2$, $R_3$, $R_4$, $R_5$, $R_6$, $R_9$, X, Y and A are as defined above and G is a protecting group, with a suphonylating agent containing the group:

$R_8SO_2$-

wherein $R_8$ is as defined above, and if required removing the protecting group G to give a compound of Formula (I) wherein $R_1$ is hydrogen;

or

ii) reacting a compound of formula

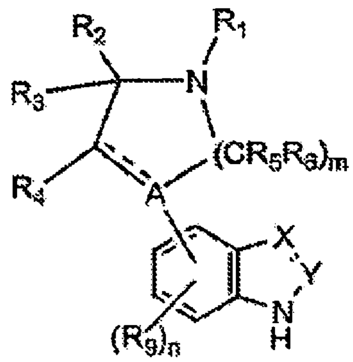

wherein the dotted line, n, m, $R_1$, $R_2$, $R_3$, $R_4$, $R_5$, $R_6$, $R_9$, X, Y and A are as defined above, with a sulphonylating agent containing the group

$R_8SO_2$-

wherein $R_8$ is as defined above, to give a compound of formula (I);

or

iii) reacting a compound of formula (I) wherein $R_1$ is hydrogen with a compound of formula:

$R_1$-L

wherein $R_1$ is as defined above (excepting hydrogen) and L is a suitable leaving group, e.g. halogen or SMe to give a corresponding compound of formula (I);

or

iv) alkylating a compound of formula (I) wherein A is $CR_{10}$ in which $R_{10}$ is OH with an alkylating agent containing the group $R_a$ where $R_a$ is optionally substituted alkyl to give a compound of formula (I) wherein $R_{10}$ is optionally substituted alkoxy;

or

v) converting a compound of formula (I) having a reactive substituent group to a different compound of formula (I).

**Patentansprüche**

1. Verbindung der Formel (I)

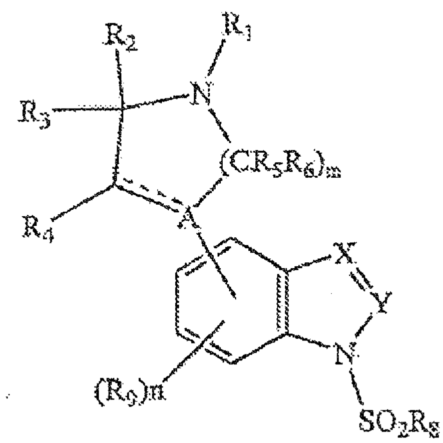

(I)

worin
A C, $CR_{10}$ oder N ist;
X N ist;
Y $CR_7$ ist;
$R_1$ H, $C_1$-$C_6$-Alkylcarbonyl, $C_1$-$C_6$-Alkoxycarbonyl oder eine $C_1$-$C_6$-Alkyl-, $C_2$-$C_6$-Alkenyl-, $C_2$-$C_6$-Alkinyl- oder Cycloheteroalkylgruppe, von denen jede gegebenenfalls substituiert ist, ist;
$R_2$, $R_3$, $R_4$, $R_5$ und $R_6$ jeweils unabhängig H, Halogen, OH oder eine gegebenenfalls substituierte $C_1$-$C_6$-Alkylgruppe sind;
$R_7$ H, Halogen oder eine $C_1$-$C_6$-Alkyl-, Aryl-, Heteroaryl- oder $C_1$-$C_6$-Alkoxygruppe, von denen jede gegebenenfalls substituiert ist, ist;
$R_8$ eine $C_1$-$C_6$-Alkyl-, Aryl- oder Heteroarylgruppe, von denen jede gegebenenfalls substituiert ist, ist;
$R_9$ H, Halogen oder eine $C_1$-$C_6$-Alkyl-, $C_1$-$C_6$-Alkoxy-, $C_2$-$C_6$-Alkenyl-, Aryl- oder Heteroarylgruppe, von denen jede gegebenenfalls substituiert ist, ist;
$M_{10}$ H, OH oder eine gegebenenfalls substituierte $C_1$-$C_6$-Alkoxygruppe ist;
m eine ganze Zahl 1, 2 oder 3 ist;
n 0 oder eine ganze Zahl 1, 2 oder 3 ist und
eine Einfachbindung oder eine Doppelbindung darstellt, oder
ein pharmazeutisch verträgliches Salz davon.

2. Verbindung gemäß Anspruch 1, wobei A N ist und m 2 ist.

3. Verbindung gemäß Anspruch 1 oder Anspruch 2, wobei $R_8$ eine gegebenenfalls substituierte Phenylgruppe ist.

4. Verbindung gemäß einem der Ansprüche 1 bis 3, wobei $R_2$, $R_3$, $R_4$, $R_5$ und $R_6$ H sind.

5. Verbindung gemäß einem der Ansprüche 1 bis 4, wobei $R_1$ H oder eine $C_1$-$C_6$-Alkyl- oder Cycloheteroalkylgruppe, von denen jede gegebenenfalls substituiert ist, ist.

6. Verbindung gemäß einem der Ansprüche 1 bis 5 zur Verwendung bei der Behandlung einer Störung des Zentralnervensystems, die mit dem 5-HT6-Rezeptor in Beziehung steht oder durch diesen beeinflusst wird.

7. Verbindung zur Verwendung gemäß Anspruch 6, wobei die Störung eine motorische Störung, Angststörung oder kognitive Störung ist.

**8.** Verbindung zur Verwendung gemäß Anspruch 6, wobei die Störung Schizophrenie oder Depression ist.

**9.** Verbindung zur Verwendung gemäß Anspruch 6, wobei die kognitive Störung eine neurodegenerative Störung ist.

**10.** Verbindung zur Verwendung gemäß Anspruch 9, wobei die neurodegenerative Störung Alzheimersche Krankheit oder Parkinsonsche Krankheit ist.

**11.** Pharmazeutische Zusammensetzung, die einen pharmazeutisch verträglichen Träger und eine Verbindung der Formel I oder ein pharmazeutisch verträgliches Salz davon, wie in einem der Ansprüche 1 bis 5 beansprucht, umfasst.

**12.** Verfahren zur Herstellung einer Verbindung der Formel (I) :

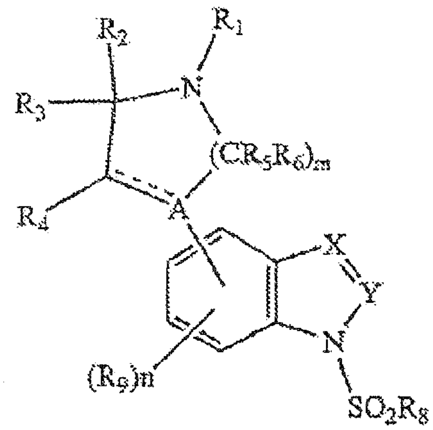

(I)

worin
A C, $CR_{10}$ oder N ist;
X N ist;
Y $CR_7$ ist;
$R_1$ H, $C_1$-$C_6$-Alkylcarbonyl, ($C_1$-$C_6$-Alkoxy) carbonyl oder eine $C_1$-$C_6$-Alkyl-, $C_2$-$C_6$-Alkenyl-, $C_2$-$C_6$-Alkinyl- oder Cycloheteroalkylgruppe, von denen jede gegebenenfalls substituiert ist, ist;
$R_2$, $R_3$, $R_4$, $R_5$ und $R_6$ jeweils unabhängig H, Halogen, OH oder eine gegebenenfalls substituierte $C_1$-$C_6$-Alkylgruppe sind;
$R_7$ H, Halogen oder eine $C_1$-$C_6$-Alkyl-, Aryl-, Heteroaryl- oder $C_1$-$C_6$-Alkoxygruppe, von denen jede gegebenenfalls substituiert ist, ist;
$R_8$ eine $C_1$-$C_6$-Alkyl-, Aryl- oder Heteroarylgruppe, von denen jede gegebenenfalls substituiert ist, ist;
Rg H, Halogen oder eine $C_1$-$C_6$-Alkyl-, $C_1$-$C_6$-Alkoxy-, $C_2$-$C_6$-Alkenyl-, Aryl- oder Heteroarylgruppe, von denen jede gegebenenfalls substituiert ist, ist;
$R_{10}$ H, OH oder eine gegebenenfalls substituierte $C_1$-$C_6$-Alkoxygruppe ist;
m eine ganze Zahl 1, 2 oder 3 ist;
n 0 oder eine ganze Zahl 1, 2 oder 3 ist und
------ eine Einfachbindung oder eine Doppelbindung darstellt;
wobei das Verfahren eines der folgenden umfasst:

i) Umsetzen einer Verbindung der Formel:

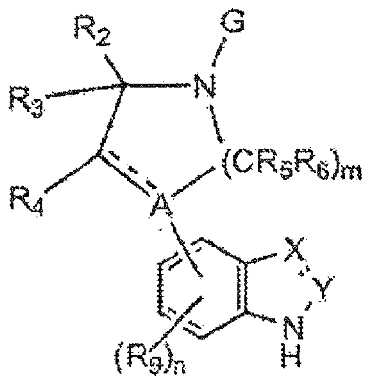

worin die gepunktete Linie, n, m, $R_2$, $R_3$, $R_4$, $R_5$, $R_6$, $R_9$, X, Y und A wie oben definiert sind und G eine Schutzgruppe ist, mit einem Sulphonylierungsmittel, das die Gruppe

$R_8SO_2$-

enthält, worin $R_8$ wie oben definiert ist, und, falls erforderlich Entfernen der Schutzgruppe G unter Erhalt einer Verbindung der Formel (I), worin $R_1$ Wasserstoff ist;
oder
ii) Umsetzen einer Verbindung der Formel

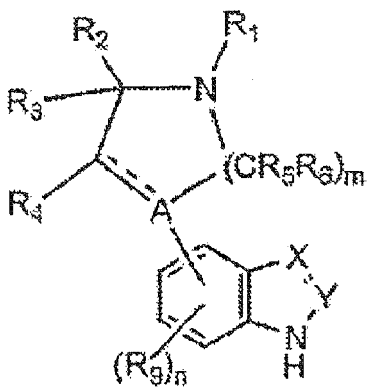

worin die gestrichelte Linie, n, m, $R_1$, $R_2$, $R_3$, $R_4$, $R_5$, $R_6$, $R_9$, X, Y und A wie oben definiert sind, mit einem Sulphonylierungsmittel, das die Gruppe

$R_8SO_2$-

enthält, worin $R_8$ wie oben definiert ist, unter Erhalt einer Verbindung der Formel (I);
oder
iii) Umsetzen einer Verbindung der Formel (I), worin $R_1$ Wasserstoff ist, mit einer Verbindung der Formel:

$R_1$-L

worin $R_1$ wie oben definiert ist (ausgenommen Wasserstoff) und L eine geeignete Abgangsgruppe, zum Beispiel Halogen oder SMe ist, um eine entsprechende Verbindung der Formel (I) zu ergeben;
oder
iv) Alkylieren einer Verbindung der Formel (I), worin A $CR_{10}$ ist, worin $R_{10}$ OH ist, mit einem Alkylierungsmittel, das die Gruppe $R_a$ enthält, worin $R_a$ gegebenenfalls substituiertes Alkyl ist, um eine Verbindung der Formel (I) zu ergeben, worin $M_{10}$ gegebenenfalls substituiertes Alkoxy ist;

oder
v) Umwandeln einer Verbindung der Formel (I) mit einer reaktiven Substituentengruppe zu einer anderen Verbindung der Formel (I).

## Revendications

**1.** Composé de formule (I)

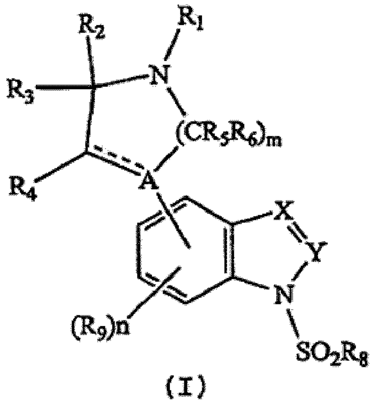

(I)

dans lequel
A représente C, $CR_{10}$ ou N ;
X représente N ;
Y représente $CR_7$ ;
$R_1$ représente H, (alkyle en $C_1$-$C_6$)carbonyle, (alcoxy en $C_1$-$C_6$) carbonyle ou un groupe alkyle en $C_1$-$C_6$, alkényle en $C_2$-$C_6$, alkynyle en $C_2$-$C_6$ ou cyclohétéroalkyle, chacun étant facultativement substitué ;
$R_2$, $R_3$, $R_4$, $R_5$ et $R_6$ représentent chacun indépendamment H, un halogène, OH ou un groupe alkyle en $C_1$-$C_6$ facultativement substitué ;
$R_7$ représente H, un halogène ou un groupe alkyle en $C_1$-$C_6$, aryle, hétéroaryle ou alcoxy en $C_1$-$C_6$, chacun étant facultativement substitué ;
$R_8$ représente un groupe alkyle en $C_1$-$C_6$, aryle ou hétéroaryle, chacun étant facultativement substitué ;
$R_9$ représente H, un halogène ou un groupe alkyle en $C_1$-$C_6$, alcoxy en $C_1$-$C_6$, alkényle en $C_2$-$C_6$, aryle ou hétéroaryle, chacun étant facultativement substitué ;
$R_{10}$ représente H, OH ou un groupe alcoxy en $C_1$-$C_6$ facultativement substitué ;
m est un entier consistant en 1, 2 ou 3 ;
n représente 0 ou un entier consistant en 1, 2 ou 3 ; et
------ représente une liaison simple ou une double-liaison ;
ou sel pharmaceutiquement acceptable correspondant.

**2.** Composé selon la revendication 1, dans lequel A représente N et m représente 2.

**3.** Composé selon la revendication 1 ou la revendication 2, dans lequel $R_8$ est un groupe phényle facultativement substitué.

**4.** Composé selon l'une quelconque des revendications 1 à 3, dans lequel $R_2$, $R_3$, $R_4$, $R_5$ et $R_6$ représentent H.

**5.** Composé selon l'une quelconque des revendications 1 à 4, dans lequel $R_1$ représente H ou un groupe alkyle en $C_1$-$C_6$ ou cyclohétéroalkyle, chacun étant facultativement substitué.

**6.** Composé selon l'une quelconque des revendications 1 à 5, pour une utilisation dans le traitement d'un trouble du système nerveux central lié au, ou affecté par le, récepteur 5-HT6.

**7.** Composé destiné à une utilisation selon la revendication 6, dans lequel ledit trouble est un trouble moteur, un trouble

d'anxiété ou un trouble cognitif.

**8.** Composé destiné à une utilisation selon la revendication 6, dans lequel ledit trouble est la schizophrénie ou la dépression.

**9.** Composé destiné à une utilisation selon la revendication 6, dans lequel ledit trouble cognitif est un trouble neurodégénératif.

**10.** Composé destiné à une utilisation selon la revendication 9, dans lequel ledit trouble neurodégénératif est la maladie d'Alzheimer ou la maladie de Parkinson.

**11.** Composition pharmaceutique comprenant un support pharmaceutiquement acceptable et un composé de formule I ou un sel pharmaceutiquement acceptable correspondant, tel que revendiqué dans l'une quelconque des revendications 1 à 5.

**12.** Procédé de préparation d'un composé de formule (I) :

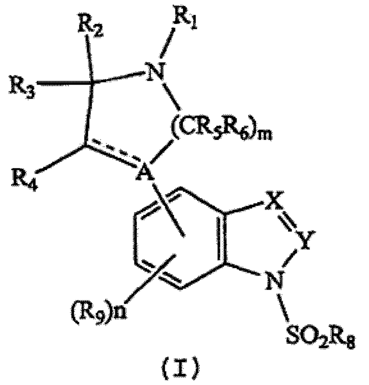

(I)

dans lequel
A représente C, $CR_{10}$ ou N ;
X représente N ;
Y représente $CR_7$ ;
$R_1$ représente (alkyle en $C_1$-$C_6$) carbonyle, (alcoxy en $C_1$-$C_6$) carbonyle ou un groupe alkyle en $C_1$-$C_6$, alkényle en $C_2$-$C_6$, alkynyle en $C_2$-$C_6$ ou cyclohétéroalkyle, chacun étant facultativement substitué ;
$R_2$, $R_3$, $R_4$, $R_5$ et $R_6$ représentent chacun indépendamment H, un halogène, OH ou un groupe alkyle en $C_1$-$C_6$ facultativement substitué ;
$R_7$ représente H, un halogène ou un groupe alkyle en $C_1$-$C_6$, aryle, hétéroaryle ou alcoxy en $C_1$-$C_6$, chacun étant facultativement substitué ;
$R_8$ représente un groupe alkyle en $C_1$-$C_6$, aryle ou hétéroaryle, chacun étant facultativement substitué ;
$R_9$ représente H, un halogène ou un groupe alkyle en $C_1$-$C_6$, alcoxy en $C_1$-$C_6$, alkényle en $C_2$-$C_6$, aryle ou hétéroaryle, chacun étant facultativement substitué ;
$R_{10}$ représente H, OH ou un groupe alcoxy en $C_1$-$C_6$ facultativement substitué ;
m est un entier consistant en 1, 2 ou 3 ;
n représente 0 ou un entier consistant en 1, 2 ou 3 ; et
------ représente une liaison simple ou une double-liaison ;
ledit procédé comprenant l'une des réactions suivantes :

i) la réaction d'un composé de formule :

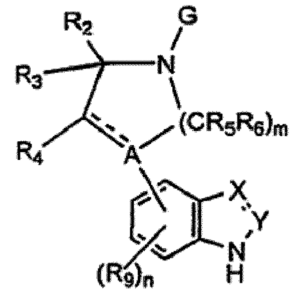

dans lequel la ligne en pointillés, n, m, $R_2$, $R_3$, $R_4$, $R_5$, $R_6$, $R_9$, X, Y et A sont tels que définis ci-dessus et G est un groupe protecteur, avec un agent sulfonylant contenant le groupe :

$R_8SO_2$-

dans lequel $R_8$ est tel que défini ci-dessus, et, si nécessaire, l'élimination du groupe protecteur G pour obtenir un composé de Formule (I) dans lequel $R_1$ représente de l'hydrogène ;
ou
ii) la réaction d'un composé de formule

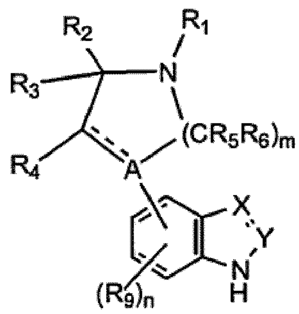

dans lequel la ligne en pointillés, n, m, $R_1$, $R_2$, $R_3$, $R_4$, $R_5$, $R_6$, $R_9$, X, Y et A sont tels que définis ci-dessus, avec un agent sulfonylant contenant le groupe

$R_8SO_2$-

dans lequel $R_8$ est tel que défini ci-dessus, pour obtenir un composé de formule (I) ;
ou
iii) la réaction d'un composé de formule (I), dans lequel $R_1$ représente de l'hydrogène, avec un composé de formule :

$R_1$-L

dans lequel $R_1$ est tel que défini ci-dessus (à l'exception de l'hydrogène) et L est un groupe labile convenable, par exemple un halogène ou SMe, pour obtenir un composé de formule (I) correspondant ;
ou
iv) l'alkylation d'un composé de formule (I) dans lequel A représente $CR_{10}$, $R_{10}$ représentant OH, avec un agent alkylant contenant le groupe $R_a$, $R_a$ représentant alkyle facultativement substitué, pour obtenir un composé de formule (I) dans lequel $R_{10}$ représente alcoxy facultativement substitué ;
ou
v) la transformation d'un composé de formule (I) ayant un groupe de substitution réactif en un composé de formule (I) différent.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Lowry et al.** *J. Biol. Chem.,* 1951, vol. 193 (265